# EUROPEAN PATENT SPECIFICATION

(11) **EP 1 437 999 B1**
(45) Date of publication and mention of the grant of the patent: **20.12.2006**
(21) Application number: 02781286.6
(22) Date of filing: 25.10.2002
(51) Int. Cl.: A61K 6/00, A61K 6/083

(54) **HYDROLYSIS STABLE SELF-ETCHING, SELF-PRIMING ADHESIVE**
HYDROLYSESTABILE SELBSTÄTZENDER, SELBSTGRUNDIERENDER KLEBSTOFF
ADHESIF STABLE A L'HYDROLYSE CONSTITUANT UN PRIMER ET UN AUTO-MORDEN AGE

(30) Priority: 26.10.2001 US 345994 P
(43) Date of publication of application: 21.07.2004
(73) Proprietor: DENTSPLY DETREY GmbH, 78467 Konstanz (DE)
(72) Inventor: KLEE, Joachim, 78315 Radolfzell (DE); WALZ, Uwe, 78465 Konstanz (DE); LEHMANN, Uwe, 78467 Konstanz (DE)
(74) Representative: Hartz, Nikolai
(86) International application number: PCT/EP2002/011940
(87) International publication number: WO 2003/035013

(56) References cited:
- EP-A- 0 705 590
- EP-A- 0 811 368
- EP-A- 1 057 468
- EP-A- 1 148 060
- WO-A-02/02057
- DD-A- 295 645
- US-A- 4 539 382
- US-B1- 6 174 935

## Description

### Technical field of the invention

The invention relates to dental adhesive compositions for bonding dental restoratives to dentin and/or enamel. More specifically the invention provides a one-part self-etching, self-priming dental adhesive composition comprising hydrolysis stable polymerizable acidic adhesive monomers.

### Background art of the invention

Omura et al in US 4,539,382 disclose two part adhesives. Moszner et al. in CA 2250333 (DE 19746708 and EP 0909761) disclose hydrolysis stable monomers. Loehden et al. in DE 19918974 disclose polymerizable phosphonic esters. Haberland in DD 273846 discloses polymerizable phosphonic amides. Two-part self-etching, self-priming dental adhesive systems are either applied sequentially or in one step after mixing the two parts. Both procedures have inherent disadvantages due to clinical complications which might occur in-between sequential steps (saliva or blood contamination) or due to dosing problems when mixing is required prior to the application of the self-etching adhesive. In order to overcome these clinical problems it would be advantageous to provide the self-etching adhesive as a one-part system eliminating the need of sequential application or premixing.

US 6,174,935 describes a dental adhesive kit containing a polymerizable N-substituted acrylic acid amide monomer having a sulfonic acid moiety. EP 1 057 468 A1 describes an adhesive composition containing an acid-group containing polymerizable monomer. WO 02/02057 describes a dental material containing phosphonic acids.

### Description of the invention

The present invention relates to a hydrolysis stable one-part self-etching, self-priming dental adhesive as defined by claim 1.

In order to increase each effect and adhesion an organic and/or inorganic acid such as methacrylic acid, acrylic acid, fumaric acid, maleic acid, citric acid, itaconic acid may be added to the hydrolysis stable one-part self-etching, self-priming dental adhesive. Preferred organic water soluble solvents may be selected from the group of alcohols and ketones, such as ethanol, propanol, butanol, acetone, methyl ethyl ketone.

Preferred polymerizable (meth) acrylamides that comprise at least a phosphonic or sulfonic acid moiety for use in the hydrolysis stable one-part self-etching, self-priming dental adhesive composition of the invention are within the scope of the following formulas: 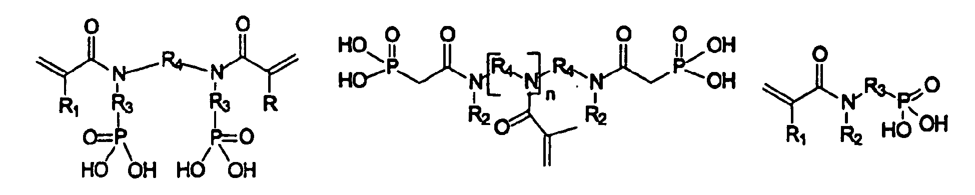 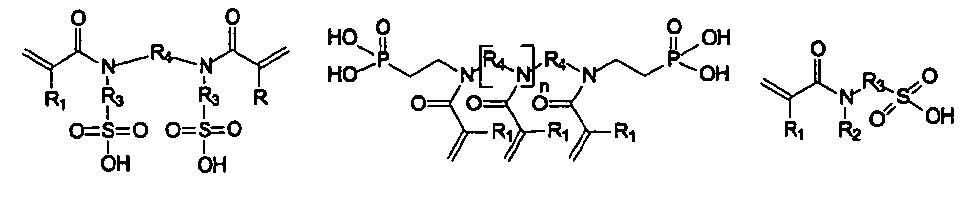 wherein
- R₁ and R₂: independently are hydrogen or a substituted or unsubstituted C₁ to C₁₈ alkylene, substituted or unsubstituted cycloalkylene, substituted or unsubstituted C₅ to C₁₈ arylene or heteroarylene, substituted or unsubstituted C₅ to C₁₈ alkylarylene or alkylheteroarylene, substituted or unsubstituted C₇ to C₃₀ alkylene arylene,
- R₃ and R₄: independently are a difunctional substituted or unsubstituted C₁ to C₁₈ alkylene, difunctional substituted or unsubstituted cycloalkylene, difunctional substituted or unsubstituted C₅ to C₁₈ arylene or heteroarylene, difunctional substituted or unsubstituted C₅ to C₁₈ alkylarylene or alkylheteroarylene, difunctional substituted or unsubstituted C₇ to C₃₀ alkylene arylene,
- n: is an integer.

Preferred (meth)acrylamides for use in the hydrolysis stable one-part self-etching, self-priming dental adhesive composition of the invention include bis- and mono (meth) acrylamides within the scope of the following formulas:

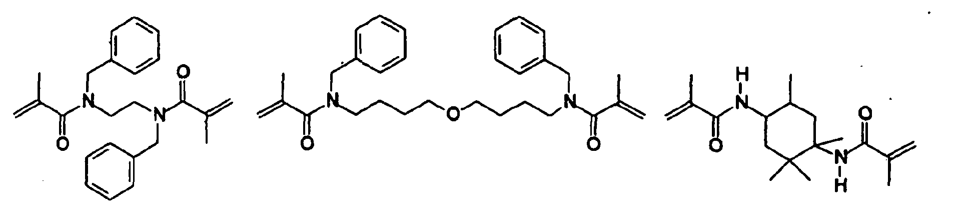

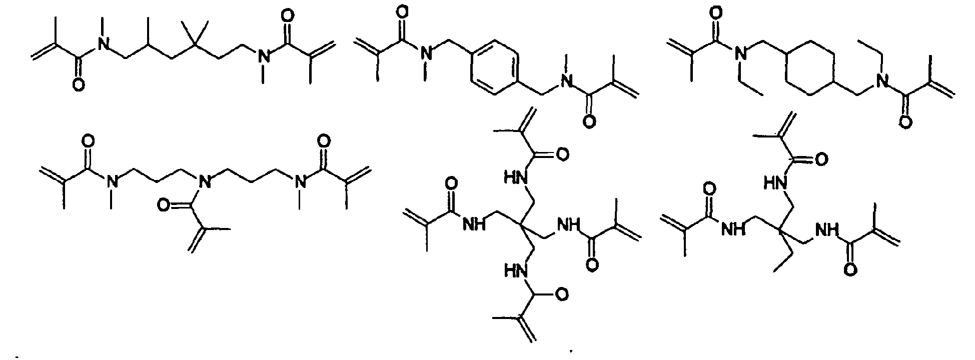

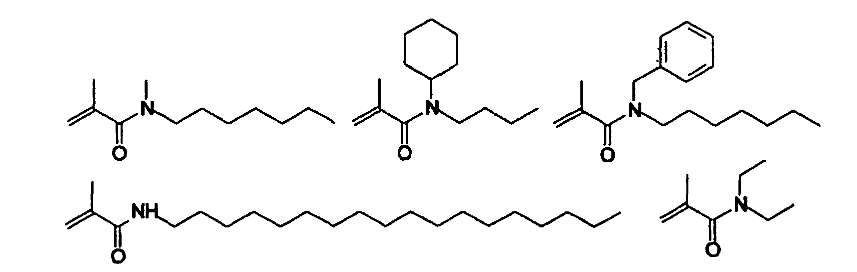

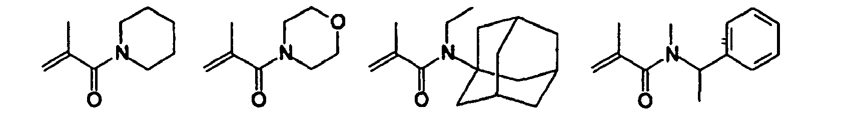

The hydrolysis stable one-part self-etching, self-priming dental adhesive composition of the invention preferably includes polymerizable hydrolysis stable monomers within the scope of the following formulas: 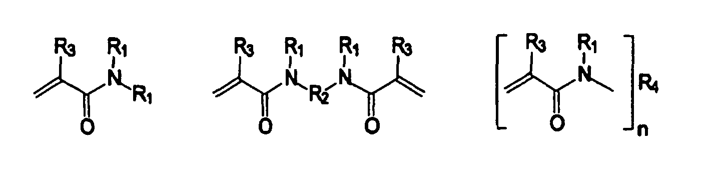 wherein
- R₁ and R₃: independently are H or a substituted or unsubstituted C₁ to C₁₈ alkylene, substituted or unsubstituted cycloalkylene, substituted or unsubstituted C₅ to C₁₈ arylene or heteroarylene, substituted or unsubstituted C₅ to C₁₈ alkylarylene or alkylheteroarylene, substituted or unsubstituted C₇ to C₃₀ alkylene arylene,
- R₂: is a difunctional substituted or unsubstituted C₁ to C₁₈ alkylene, difunctional substituted or unsubstituted cycloalkylene, difunctional substituted or unsubstituted C₅ to C₁₈ arylene or heteroarylene, difunctional substituted or unsubstituted C₅ to C₁₈ alkylarylene or alkylheteroarylene, difunctional substituted or unsubstituted C₇ to C₃₀ alkylene arylene,
- R₄: is a mono- or polyfunctional substituted or unsubstituted C₁ to C₁₈ alkylene, mono- or polyfunctional substituted or unsubstituted cycloalkylene, mono- or polyfunctional substituted or unsubstituted C₅ to C₁₈ arylene or heteroarylene, mono- or polyfunctional substituted or unsubstituted C₅ to C₁₈ alkylarylene or alkylheteroarylene, mono- or polyfunctional substituted or unsubstituted C₇ to C₃₀ alkylene arylene,
- n: is an integer.

The compositions of the invention preferably include at least a bis- or poly (meth)acrylamide, a polymerizable mono acrylamide, an initiator, a stabilizer, water and/or an organic solvent. The polymerization initiator is perferably a thermal initiator, a redox-initiator or a photo initiator preferably used is champhor quinone. To stabilize the dental composition a stabilizer may be included which absorbs radicals, such as hydroquinone monomethylether, 2,6-di-tert.-butyl-p-cresol, tetramethyl piperidine N-oxyl radical, galvanoxyl radical.

Preferably the hydrolysis stable one-part self-etching, self-priming dental adhesive of the invention includes from 5 to 95 percent by weight of hydrolysis stable polymerizable monomer, and from 0.01 to 30 percent by weight of organic and/or inorganic acid.

### Best mode for carrying out the invention

The present invention provides an aqueous one-pack self-etching and self-priming dental adhesive composition having a pH of at most 2, as defined by claim 1.

A one-pack composition means that the composition of the present invention is contained in only one container which may be stored and allows application of the composition without any mixing and without any special equipment before the application.

Self-etching means that the dental adhesive composition of the present invention may be applied to a tooth without any preliminarily etching of enamel in a separate method step. In order to comprise such a self-etching feature, the composition of the present invention is aqueous and has a pH of at most 2. Preferably the pH is below 2, more preferably the pH is below 1.5, most preferably the pH is about 1. An etching of enamel is thus advantageously achieved with the one-pack composition of the invention. It allows adhesion of an adhesive prepared from the dental composition to enamel and/or dentin with a bond strength of at least 8 MPa, preferably at least 10 MPa.

Self-priming means that the dental adhesive composition of the present invention may be applied to a tooth without any preliminarily application of a primer.

The polymerizable N-substituted alkylacrylic or acrylic acid amide monomer which optionally contains an inorganic acidic moiety selected from a phosphonic acid moiety or a sulfonic acid moiety has preferably one of the following structures: 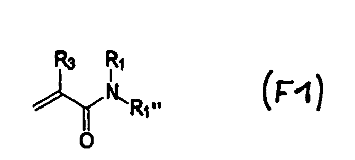 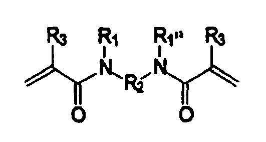 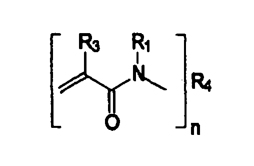 wherein
R₁, R_{1"} and R₃ independently are hydrogen or a substituted or unsubstituted C₁ to C₁₈ alkyl group, a substituted or unsubstituted cycloalkyl group, a substituted or unsubstituted C₅ to C₁₈ aryl or heteroaryl group, a substituted or unsubstituted C₅ to C₁₈ alkylaryl or alkylheteroaryl group, a substituted or unsubstituted C₇ to C₃₀ aralkyl group,
provided that in case one of R₁ and R_{1"}. is hydrogen in formula (F1) the other is not hydrogen;
R₂ is a difunctional substituted or unsubstituted C₁ to C₁₈ alkylene, difunctional substituted or unsubstituted cycloalkylene, a difunctional substituted or unsubstituted C₅ to C₁₈ aryl or heteroaryl group, a difunctional substituted or unsubstituted C₅ to C₁₈ alkylaryl or alkylheteroaryl group, a difunctional substituted or unsubstituted C₇ to C₃₀ aralkyl group,
R₄ is a mono- or polyfunctional substituted or unsubstituted C₁ to C₁₈ carbon chain group, a mono- or polyfunctional substituted or unsubstituted cycloalkyl group, a mono- or polyfunctional substituted or unsubstituted C₅ to C₁₈ aryl or heteroaryl group, a mono- or polyfunctional substituted or unsubstituted C₅ to C₁₈ alkylaryl or alkylheteroaryl group, a mono- or polyfunctional substituted or unsubstituted C₇ to C₃₀ aralkyl group, and
n is an integer, preferably from 1 to 10, more preferably from 3 to 4.

The optional substituents on the groups represented by R₁, R_{1"}, R₂, R₃, and R₄ are preferably selected from C₁ to C₁₈ alkyl groups. Particularly preferred are C₁ to C₆ alkyl groups, whereby methyl groups are especially preferred.

Preferred is that R₂ and R₄ independently is a di- or polyfunctional substituted or unsubstituted C₁ to C₁₈ carbon chain group or a di- or polyfunctional substituted or unsubstituted cycloalkylene group, which has at least one linkage of ether, thioether, ester, thiocarbonyl, amide, carbonyl, sulfonyl, urethane, or substituted or unsubstituted amine linkages.

A C₁ to C₁₈ carbon chain group means a branched or straight hydrocarbon having at least two bonds or valences and from 1 to 18 carbon atoms. In case of only 1 carbon atom the C₁ carbon chain group may have from 2 to 4 bonds or valences and from 2 to 0 hydrogen substituents, i.e. the C₁ carbon chain group may have one of the following structures:

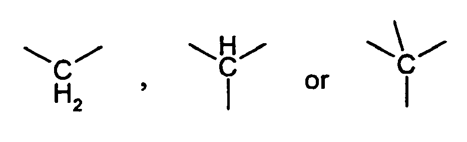

Particularly preferred is that R₂ or R₄ is -(CH₂)₂-O-(CH₂)₂-O-(CH₂)₂-, 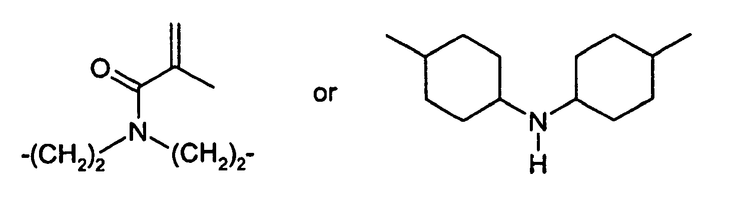 and n is 2.

In a preferred embodiment of the present invention the polymerizable N-substituted alkylacrylic or acrylic acid amide monomer which optionally contains an inorganic acidic moiety selected from a phosphonic acid moiety or a sulfonic acid moiety is selected from the-group consisting of compounds represented by the following formulas: 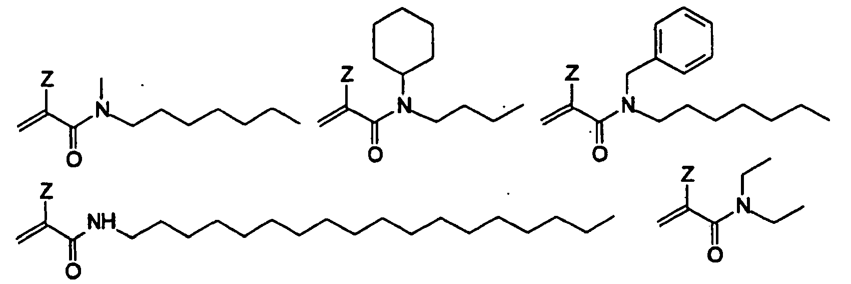 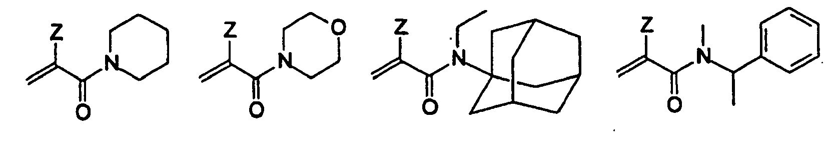 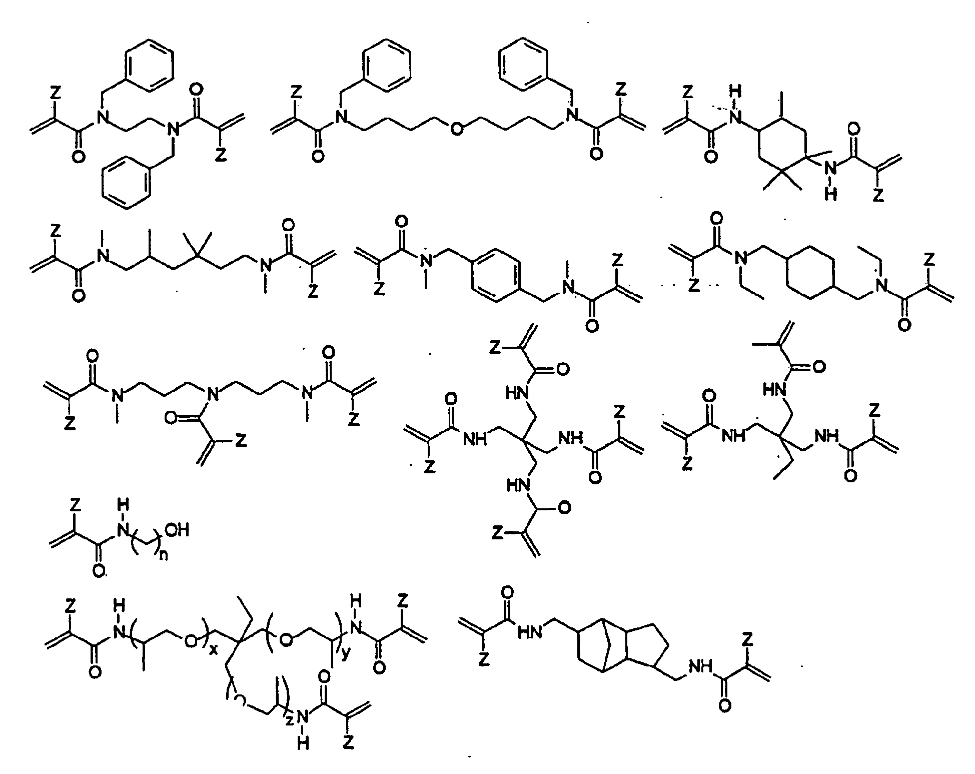 wherein
n is an integer of from 2 to 6;
x, y, and z independently is an integer of from 1 to 10; and
Z is H or a C₁ to C₁₈ alkyl group. The formula having x, y, z is a mixture of compounds. Particularly preferred is a mixture, wherein x+y+z is 5.3.

In a preferred embodiment of the present invention, the polymerizable N-substituted alkylacrylic or acrylic acid amide monomer which optionally contains an inorganic acidic moiety selected from a phosphonic acid moiety or a sulfonic acid moiety is selected from (meth)acrylamide monomers, preferably from the type of secondary amides, since these are particularly hydrolysis stable.

In a preferred embodiment of the present invention the polymerizable N-substituted alkylacrylic or acrylic acid amide monomer which optionally contains an inorganic acidic moiety selected from a phosphonic acid moiety or a sulfonic acid moiety contains at least such an inorganic acidic moiety, preferably a phosphonic acid moiety or a sulfonic acid moiety: Preferably, the groups represented by R₁, R_{1''} , R₂, R₃, and R₄ as described above may be substituted by a group containing at least an inorganic acidic moiety, preferably a phosphonic acid moiety or a sulfonic acid moiety. Particularly preferred is that R₂ and R₄ contain such an inorganic acidic moiety. Thus, no separate acid has to be incorporated into the composition of the present invention to obtain a pH of at most 2. Then the polymerizable N-substituted alkylacrylic or acrylic acid amide monomer which optionally contains an inorganic acidic moiety selected from a phosphonic acid moiety or a sulfonic acid moiety may be represented by the the polymerizable N-substituted alkylacrylic or acrylic acid amide monomer containing at least an acidic moiety as described below.

However, the composition of the present invention may comprise an organic or inorganic acid, whereby the organic acid is selected from the group consisting of methacrylic acid, acrylic acid, fumaric acid, maleic acid, citric acid, itaconic acid, and formic acid and whereby the inorganic acid is selected from phosphoric acid, sulfuric acid and hydrofluoric acid. The incorporation of an acid is necessary in case the polymerizable N-substituted alkylacrylic or acrylic acid amide monomer which optionally contains an inorganic acidic moiety selected from a phosphonic acid moiety or a sulfonic acid moiety does not contain an acidic moiety.

However, in a further embodiment of the present invention the composition of the present invention further comprises
(iii) a polymerizable N-substituted alkylacrylic or acrylic acid amide monomer containing at least an inorganic acidic moiety selected from a phosphonic acid moiety or a sulfonic acid moiety.

The polymerizable N-substituted alkylacrylic or acrylic acid amide monomer containing at least an inorganic acidic moiety is preferably selected from the group consisting of compounds represented by the following formulas: 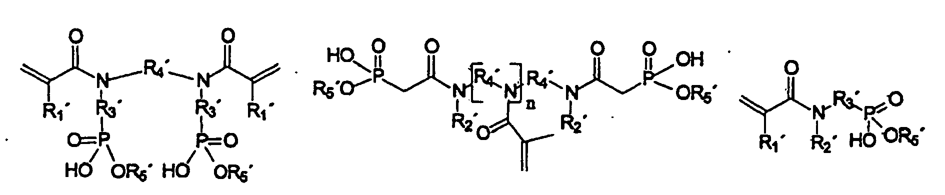 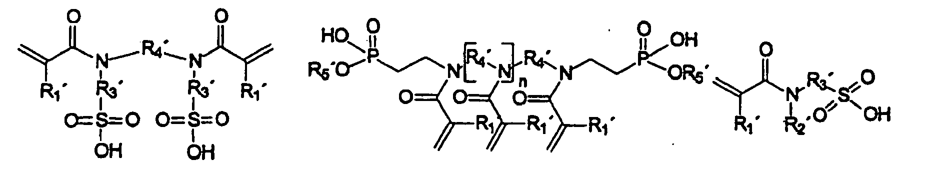 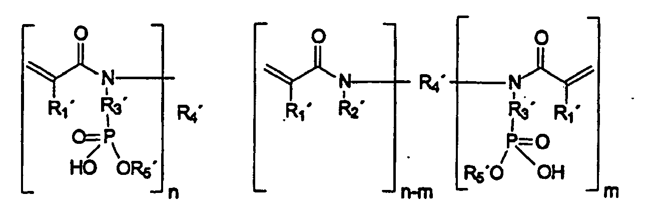 wherein
R_{1'} and R_{2'} represent independently from each other a hydrogen atom or a substituted or unsubstituted C₁ to C₁₈ alkyl group, a substituted or unsubstituted cycloalkyl group, a substituted or unsubstituted C₅ to C₁₈ aryl or heteroaryl group, a substituted or unsubstituted C₅ to C₁₈ alkylaryl or alkylheteroaryl group, a substituted or unsubstituted C₇ to C₃₀ aralkyl group,
R_{3'} and R_{4'} represent independently from each other a difunctional substituted or unsubstituted C₁ to C₁₈ carbon chain group, a difunctional substituted or unsubstituted cycloalkylene, a difunctional substituted or unsubstituted C₅ to C₁₈ aryl or heteroaryl group, a difunctional substituted or unsubstituted C₅ to C₁₈ alkylaryl or alkylheteroaryl group, a difunctional substituted or unsubstituted C₇ to C₃₀ aralkyl group,
R_{5'} represents H or a substituted or unsubstituted C₁ to C₁₈ alkyl group
n is an integer, preferably from 1 to 18 or 1 to 4 and
m is an integer, preferably from 1 to 3.

The optional substituents on the groups represented by R_{1'}, R_{2'}, R_{3'}, R_{4'} and R_{5'} are preferably selected from C₁ to C₁₈ alkyl groups. Particularly preferred are C₁ to C₆ alkyl groups, whereby methyl groups are most preferred. The C₁ to C₁₈ carbon chain group is as defined above.

Preferably, R_{3'} and R_{4'} independently from each other is a difunctional substituted or unsubstituted C₁ to C₁₈ carbon chain group or a difunctional substituted or unsubstituted cycloalkylene, which has at least one linkage of ether, thioether, ester, thiocarbonyl, amide, carbonyl, sulfonyl, urethane, or substituted or unsubstituted amine linkages.

More, preferably R₃, is -(CH₂)₂- or 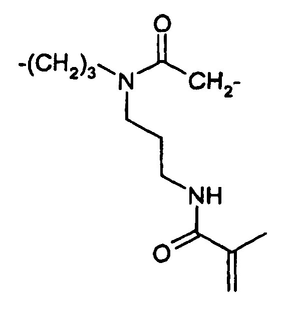 and R_{4'} is -(CH₂)₂-O-(CH₂)₂-O-(CH₂)₂-.

In a preferred embodiment of the present invention polymerizable N-substituted alkylacrylic or acrylic acid amide monomer containing at least an inorganic acidic moiety is selected from one of the monomers according to the following formulas:

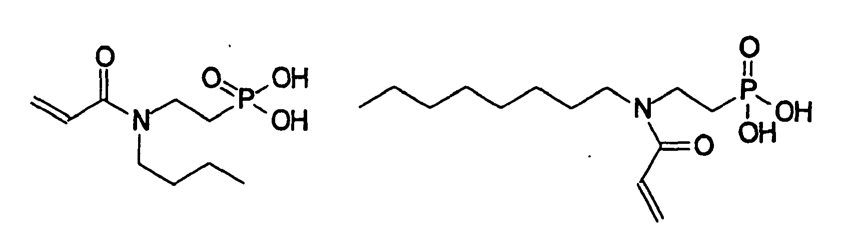

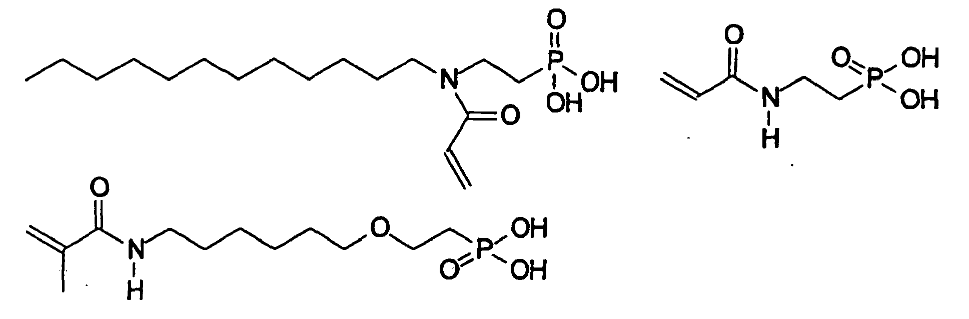

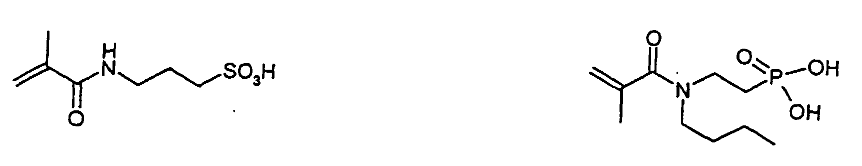

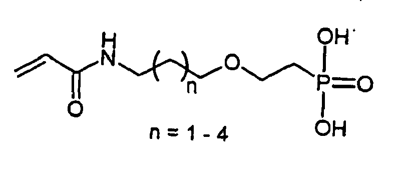

In a particular preferred embodiment of the present invention the polymerizable N-substituted alkylacrylic or acrylic acid amide monomer containing at least an inorganic acidic moiety is selected from (meth)acrylamide monomers, preferably from the type of secondary amides, more preferably from acrylamide monomers from the type of secondary amides, since these are particularly hydrolysis stable.

The polymerizable N-substituted alkylacrylic or acrylic acid amide monomer containing at least an inorganic acidic moiety selected from a phosphonic acid moiety or a sulfonic acid moiety (iii) is preferably incorporated into the composition of the present invention in case none of the above organic or inorganic acids is incorporated into the composition of the present invention and/or in case the polymerizable N-substituted alkylacrylic or acrylic acid amide monomer which optionally contains an inorganic acidic moiety selected from a phosphonic acid moiety or a sulfonic acid moiety (i) does not contain an acidic moiety. However, the polymerizable N-substituted alkylacrylic or acrylic acid amide monomer containing at least an inorganic acidic moiety selected from a phosphonic acid moiety or a sulfonic acid moiety (iii) may also be incorporated into the composition of the present invention in case the latter contains either an organic or inorganic acid or the polymerizable N-substituted alkylacrylic or acrylic acid amide monomer which optionally contains an inorganic acidic moiety selected from a phosphonic acid moiety or a sulfonic acid moiety (i) which contains an acidic moiety. Further, the polymerizable N-substituted alkylacrylic or acrylic acid amide monomer containing at least an inorganic acidic moiety selected from a phosphonic acid moiety or a sulfonic acid moiety (iii) may also be incorporated into the composition of the present invention in case both an inorganic and/or organic acid and the polymerizable N-substituted alkylacrylic or acrylic acid amide monomer which optionally contains an inorganic acidic moiety selected from a phosphonic acid moiety or a sulfonic acid moiety (i) which contains an acidic moiety are incorporated into the composition of the present invention.

Preferably, the composition of the present invention is hydrolysis stable for at least one week at a storage temperature of 50°C, whereby after such storage the bond strength of an adhesive prepared from such an adhesive composition to enamel and/or dentin is at least 8 MPa, preferably 10 MPa.

The aqueous composition of the present invention may contain beside water an organic water-soluble solvent, preferably selected from alcohols and ketones.

Preferably, the organic water-soluble solvent is selected from ethanol, propanol, butanol, acetone, and methyl ethyl ketone.

Preferably, the polymerizable N-substituted alkylacrylic or acrylic acid amide monomer which optionally contains an inorganic acidic moiety selected from a phosphonic acid moiety or a sulfonic acid moiety (i) is hydrolysis-stable according to the following test:
1.5 mmol of the polymerizable N-substituted alkylacrylic or acrylic acid amide monomer (i), and in case the monomer does not contain an acidic moiety 1 equivalent of ethanesulfonic acid per alkylacrylamide or acrylamide group, are solved in 5 g of a solvent mixture consisting of 50 wt.-% ethanol and 50 wt.-% water to obtain a mixture which is stored in a closed vial in an oven at 50°C; and
after 2 weeks of such a storage the hydrolysis of the tested monomer is less than 50% according to a HPLC-analysis of the absolute amount of the alkylacrylic or acrylic acid formed by hydrolysis of the tested monomer.

In a further preferred, the polymerizable N-substituted alkylacrylic or acrylic acid amide monomer containing at least an inorganic acidic moiety selected from a phosphonic acid moiety or a sulfonic acid moiety (iii) is hydrolysis-stable according to the following test:
1.5 mmol of the polymerizable N-substituted alkylacrylic or acrylic acid amide monomer containing at least an inorganic acidic moiety selected from a phosphonic acid moiety or a sulfonic acid moiety (iii) are solved in 5 g of a solvent mixture consisting of 50 wt.-% ethanol and 50 wt.-% water to obtain a mixture which is stored in a closed vial in an oven at 50°C; and
after 2 weeks of such a storage the hydrolysis of the tested polymerizable N-substituted alkylacrylic or acrylic acid amide monomer is less than 50% according to a HPLC-analysis of the absolute amount of the alkylacrylic or acrylic acid formed by hydrolysis of the tested polymerizable N-substituted alkylacrylic or acrylic acid amide monomer.

In a further preferred embodiment of the invention the above described monomer(s) is (are) hydrolysis-stable according to the preceding test(s), whereby after 1 week of the above described storage the hydrolysis of the tested monomer is less than 10% according to a HPLC-analysis of the absolute amount of alkylacrylic or acrylic acid formed by hydrolysis of the tested monomer.

Particularly preferred is that both of the above described monomers (i) and (iii) are hydrolysis stable according to the described tests in case both monomers (i) and (iii) are present in the composition of the invention. However, it is also possible that only one of the monomers (i) and (iii) is hydrolysis stable according to the above test(s) in case both of the monomers (i) and (iii) are present.

Although the polymerizable N-substituted alkylacrylic or acrylic acid amide monomer which optionally contains an inorganic acidic moiety selected from a phosphonic acid moiety or a sulfonic acid moiety and/or the polymerizable N-substituted alkylacrylic or acrylic acid amide monomer containing at least an inorganic acidic moiety of the present invention are hydrolysis stable according to the above described tests, hydrolysis may occur to a small degree. Accordingly, the polymerizable N-substituted alkylacrylic or acrylic acid amide monomer which optionally contains an inorganic acidic moiety selected from a phosphonic acid moiety or a sulfonic acid moiety and/or the polymerizable N-substituted alkylacrylic or acrylic acid amide monomer containing at least an inorganic acidic moiety of the present invention contains at least two polymerizable groups. Namely, in case one amide linkage is hydrolysed, the N-substituted alkylacrylic or acrylic acid amide monomer containing at least two polymerizable groups still contains at least one polymerizable group which allows polymerization. Thus, a high bond strength to enamel and/or dentin will be attained. The at least two polymerizable groups may be linked directly or indirectly. Preferably, they are linked via an amide bond which increases the stability of the composition and the bond strength of the adhesive composition to enamel or dentin.

The composition of the present invention may further contain a nanofiller.

In a further preferred embodiment of the present invention, the polymerizable N-substituted alkylacrylic or acrylic acid amide monomer containing at least an inorganic acidic moiety, contains at least two inorganic acidic moieties.

The curing system in the composition of the present invention comprises preferably a polymerization initiator, an inhibitor or stabilizer, preferably the curing system is a light-curing system.

In a further embodiment of the present invention an aqueous one-pack self-etching and self-priming dental adhesive composition is provided, which comprises:
(a) a polymerizable N-substituted alkylacrylic or acrylic acid amide monomer having at least two polymerizable moieties;
(b) a polymerizable N-substituted alkylacrylic or acrylic acid amide monomer containing at least an inorganic acidic moiety, and
(c) a curing system.
Preferably, the polymerizable N-substituted alkylacrylic or acrylic acid amide monomer having at least two polymerizable moieties (a) is selected from the group consisting of compounds represented by the following formulas: 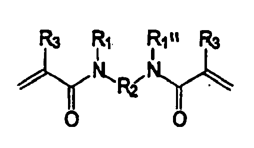 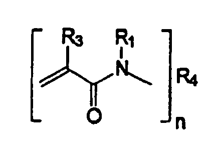 wherein
R_{1'} R_{1"}, R₂, R₃ and R₄ and n are as defined above and in claims 14 to 16.

In a particular preferred embodiment, the polymerizable N-substituted alkylacrylic or acrylic acid amide monomer having at least two polymerizable moieties (a) is selected from the group consisting of compounds represented by the following formulas: 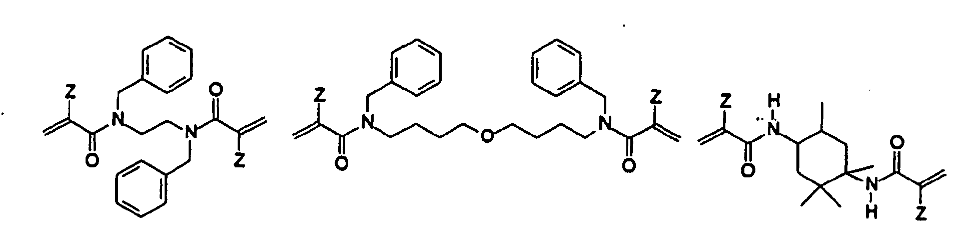 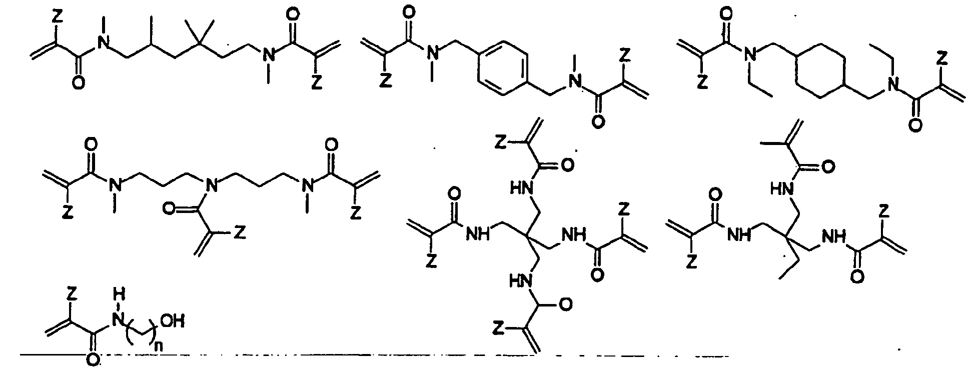 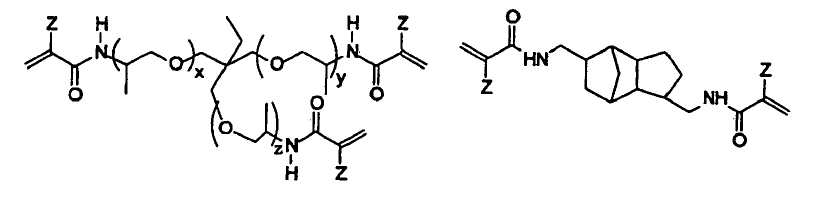 wherein Z is H or a substituted or unsubstituted C₁ to C₁₈ alkyl group and n, x, y, z are as defined above and in claim 17. Particularly preferred are (meth)acrylamide monomers, preferably from the type of secondary amides, since these are particularly hydrolysis stable.

In a further embodiment of the present invention, an aqueous one-pack self-etching and self-priming dental adhesive composition is provided which comprises:
(I) a polymerizable N-substituted alkylacrylic or acrylic acid amide monomer which optionally contains an inorganic acidic moiety selected from a phosphonic acid moiety or a sulfonic acid moiety,
(II) a polymerizable N-substituted alkylacrylic or acrylic acid amide monomer containing at least one inorganic acidic moiety, and
(III) a curing system.

Particularly preferred is that the polymerizable N-substituted alkylacrylic or acrylic acid amide monomer containing at least one inorganic acidic moiety (II) contains at least two inorganic acidic moieties.

Preferably, the polymerizable N-substituted alkylacrylic or acrylic acid amide monomer containing at least one inorganic acidic moiety (II) is selected from the group consisting of compounds represented by the following formulas having phosphonic acid moiety(ies) or sulfonic acid moiety(ies): 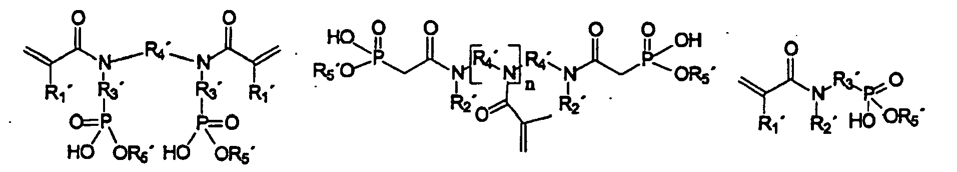 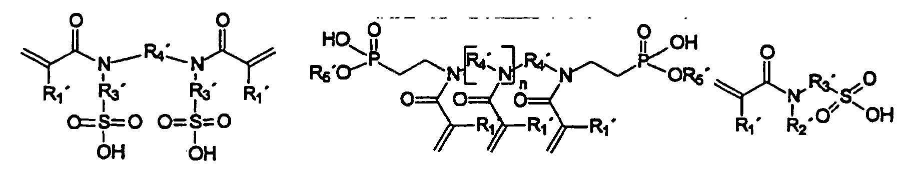 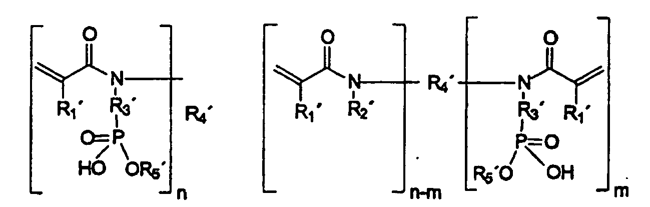 wherein
R_{1'}, R_{2'}, R_{3'}, R_{4'}, R_{5'} n and m are as defined above.

It is preferred that polymerizable N-substituted alkylacrylic or acrylic acid amide monomer containing at least one inorganic acidic moiety is selected from (meth)acrylamide monomers, preferably from the type of secondary amides, more preferably from acrylamide monomers from the type of secondary amides, since these are particularly hydrolysis-stable.

It is preferred that any composition according to the present invention is packed in a container shielded against light.

According to the present invention a polymerizable N-substituted alkylacrylic or acrylic acid amide monomer which optionally contains an inorganic acidic moiety selected from a phosphonic acid moiety or a sulfonic acid moiety and a polymerizable N-substituted alkylacrylic or acrylic acid amide monomer containing an inorganic acidic moiety are useful for the preparation of an aqueous one-pack self-etching and self-priming dental adhesive composition which comprises
(i) the polymerizable N-substituted alkylacrylic or acrylic acid amide monomer which optionally contains an inorganic acidic moiety selected from a phosphonic acid moiety or a sulfonic acid moiety,
(ii) the polymerizable N-substituted alkylacrylic or acrylic acid amide monomer containing at least an inorganic acidic moiety, and a
(iii) a curing system.

A method for the preparation of an aqueous one-pack self-etching and self-priming dental adhesive composition according to the invention is characterized by mixing
(A) a polymerizable N-substituted alkylacrylic or acrylic acid amide monomer which optionally contains an inorganic acidic moiety selected from a phosphonic acid moiety or a sulfonic acid moiety,
(B) a curing system, and
(C) a water containing solvent.

A novel dental adhesive is obtainable by polymerizing any one of the above described compositions of the present invention.

According to the present invention a method for treatment of human or animal teeth is provided, which comprises the application of the composition of one of the above described compositions.

Moreover, the present invention provides a kit comprising the composition of one of the above described compositions and instructions for use.

Furthermore, a novel monomer is provided in the present invention. It is polymerizable N-substituted alkylacrylic or acrylic acid amide monomer having at least two polymerizable moieties which is selected from the group consisting of compounds represented by the following formulas: 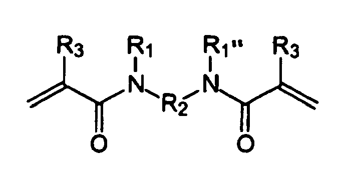 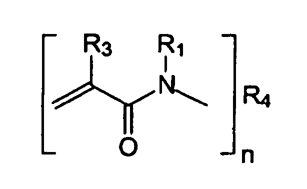 wherein
R₁, R_{1''}, R₂, R₃, R₄ and n are as defined above and in one of claims 14 to 16.

A method for the preparation of the novel monomer comprises reacting a di- or polyamine compound with an optionally substituted acryloyl halide of the formula CH₂=CR₃-CO-Hal, wherein R₃ is as defined above and in claim 14 and Hal is a halide.

A halide means chlorine, bromine, fluorine or iodine, whereby chlorine and bromine are preferred.

Further, the present invention provides a novel monomer having an acidic inorganic moiety. It is a polymerizable N-substituted alkylacrylic or acrylic acid amide monomer containing at least one inorganic acidic moiety selected from a phosphonic acid moiety or sulfonic acid moiety, which is selected from the group consisting of compounds represented by the following formulas: 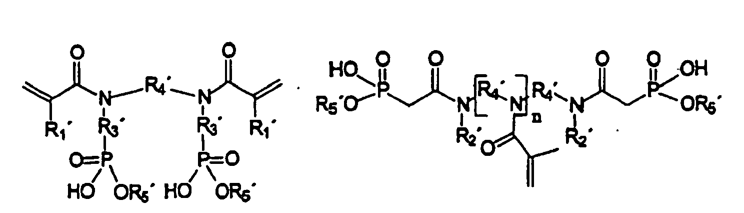 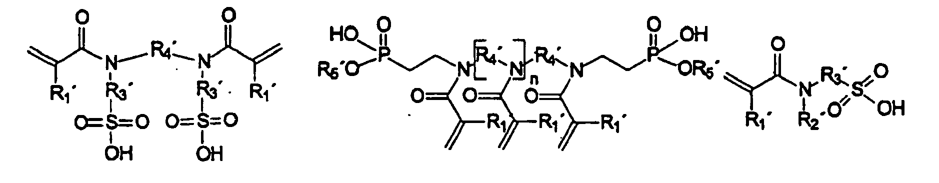 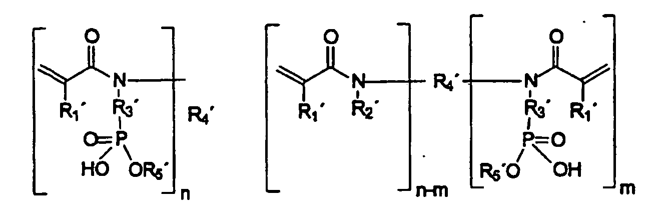 wherein
R_{1'}, R_{2'}, R_{3'}, R_{4'}, R_{5'}, n and m are as defined above.

A method for the preparation of the novel monomer having at least an inorganic acidic moiety, comprises:
(1) reacting a di- or poly amine compound with a vinylphosphonate or a vinyl sulphonic acid salt;
(2) reacting the product obtained in (1) with an alkylacryloyl or acryloyl halide of the formula CH₂=CR_{1'}-CO-Hal, wherein R_{1'} is as defined in claim 19 and Hal is a halide;
(3) optionally reacting the product obtained in (2) with a trialkyl halogensilane in case of reacting a vinyl phosphonate in step (1); and
(4) hydrolyzing the product obtained in step (2) or (3).

Particularly preferred is the monomer represented by the following formula: 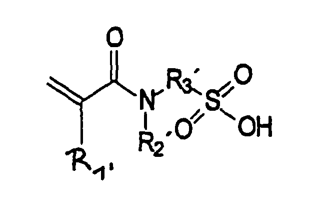 wherein R_{1'}, R_{2'} and R_{3'} are as defined above. A method for its preparation comprises:
(1) reacting an alkylacryloyl or acryloyl halide of the formula CH₂=CR₁-CO-Hal, wherein R₁ is as defined above and Hal is a halide with N-hydroxysuccinimide and an aminoalcohol of the formula HO-(CH₂)ₙ-NH₂ wherein n is from 1 to 18;
(2) reacting the product obtained in (1) or methacryloyl amide with sodium hydride and propanesulfone;
(3) hydrolyzing the product obtained in step (2).

The present invention will now be explained in further detail by the following examples, tests and application examples.

### Example 1

### N-(3-sulfopropyl) methacrylamide (1) and N,N-bis(3-sulfopropyl) methacrylamide

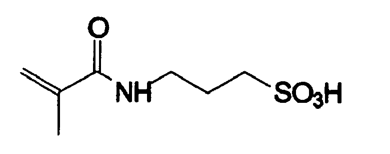

To a stirred solution of 12 g (140.9 mmol) methacrylamide in 400 ml methylene chloride 3.38 g (140.9 mmol) sodium hydride were carefully added stepwise at a temperature of 0 °C. The suspension was stirred for 3 h at room temperature before 18.943 g (155 mmol) 1,3-propanesulfone were added. After 12 d stirring at room temperature 150 ml water were dropped carefully into the reaction mixture, while the temperature was kept at 0 °C. The aqueous layer was then separated and five times extracted with 100 ml methylene chloride. Afterwards the water was removed at a rotary evaporator and the resulting white solid washed thoroughly with acetone. The sulfonic acid sodium salt was solved again in water and poured over an ion exchange column (Merck ion exchanger I). The resulting acidic aqueous solution was stabilized with 0.025 mol% hydroquinone and narrowed down at a rotary evaporator. Removal of the water under high vacuum (8 x 10⁻³mbar) afforded a mixture of N-(3-sulfopropyl) methacrylamide **1** and N,N'-bis(3-sulfopropyl) methacrylamide in a ratio of 2.6 : 1 (according to nmr spectroscopy) as a clear, reddish, highly viscose oil in an amount of 16.33 g (35% yield in regard to N-(3-sulfopropyl) methacrylamide **1**).

¹H-NMR (250 MHz, d₆-DMSO, ppm) N-(3-sulfopropyl) methacrylamide: 1.70-1.90 (m, 2H, CH₂-C*H*₂-CH₂), 1.84 (t, 3H, CH₃), 2.57-2.73 (m, 2H, C*H*₂-SO₃H), 3.36-3.47 (m, 2H, N-CH₂), 5.31 (s, 1H, CH=), 5.65 (s, 1H, CH=), 8.07 (t, 1H, NH).
¹³C-NMR (63 MHz, d₆-DMSO, ppm) N-(3-sulfopropyl) methacrylamide: 19.17 (CH₃), 25.31 (-CH₂), 38.58 (N-CH₂), 49.78 (CH₂-SO₃H), 119.76 (CH₂=), 140.35(=C-CH₃), 168.18 (C=O).

### Example 2

### N-(6-hydroxyhexyl) methacrylamide (2)

A solution of 22.56 g (0.215 mol) methacryloyl chloride in 20 ml chloroform was dropped slowly into a stirred solution of 24.84 g (0.215 mol) N-hydroxysuccinimide in 50 ml triethylamine and 500 ml chloroform at a temperature of 0 °C. After the solution was stirred for 3 h at room temperature 21.07 g (0.179 mol) 6-amino-1-hexanol in 20 ml chloroform were added. The reaction mixture was stirred overnight at room temperature before the solvent was removed at reduced pressure. The residue was taken up into methylene chloride and the remaining solid was filtered off. Then the solution was narrowed down and the precipitate filtered off again. Afterwards the solution was washed twice with 200 ml of an aqueous sodium hydroxide solution (20 %). The combined aqueous layers were washed four times with 100 ml methylene chloride and the combined organic solutions were then dried over magnesium sulfate. Filtration and evaporation of the solvent afforded a yellow oil as raw product, which was stabilized with 0.025 mol% BHT. Destillation under high vacuum (140-143 °C, 4x10-3 mbar) yielded compound 2 as a colorless, low viscose oil, which was stabilized by the addition of 0.025 mol% BHT, in an amount of 24.18 g (yield: 75%).

IR(film, cm⁻¹) 3118 (s,b), 2930 (s), 2860 (m), 1655 (s), 1611 (s), 1534 (s), 1448 (m), 1374 (w), 1318 (w), 1218 (m), 1051 (s), 925 (m).
¹H-NMR (250 MHz, CDCl₃, ppm) 1.11-1.14 (m, 4H, CH₂), 1.29-1.1.35 (m, 4H, CH₂), 1.72 (s, 3H, =C-CH₃) 3.04 (quart., 2H, CH₂-N), 3.35 (t, 2H, CH₂-O), 4.23 (broad s, 1H, OH), 5.09 (s, 1H, CH=), 5.49 (s, 1H, CH=), 6.91 (t, 1H, NH).
¹³C-NMR (63 MHz, CDCl₃, ppm) 18.16 (CH₃), 24.91, 26.15, 28.85, 31.94, 39.11 (CH₂), 61.50 (CH₂-O), 119.08 (CH₂=), 139.30 (=C-CH₃), 168.54 (C=O).

### N-[9-(diethoxyphosphoryl)-7-oxa-nonyl] methacrylamide (3)

To a solution of 11.63 g (62.8 mmol) N-(6-hydroxyhexyl) methacrylamide 2 in 250 ml methylene chloride 1.5 g (62.8 mmol) sodium hydride were added stepwise under stirring at a temperature of 0 °C. After 1 h stirring at room temperature 10.30 g (62.80 mmol) diethyl vinylphosphonate were added. The reaction mixture was stirred for additional 4 d before the reaction was terminated by the addition of 200 ml water. The layers were separated and the organic layer washed again with 100 ml water. The organic layer was dried over magnesium sulfate and filtered. Evaporation of the solvent under reduced pressure at the rotary evaporator and drying under high vacuum (8 x 10⁻³mbar) at 40 °C until the weight was constant gave 19.13 g (yield: 87%) of a yellow oil, which was stabilized with 0.025 mol% BHT.
IR(film, cm⁻¹) 3327 (m), 2932 (m), 2863 (m), 1658 (m), 1617 (m), 1525 (m), 1447 (m), 1373 (m), 1310 (w), 1222 (s), 1105 (s), 1025 (s), 957 (s), 792 (s).
¹H-NMR (250 MHz, CDCl₃, ppm) 1.06 (t. 6H, CH₃), 1.01-1.17 (m, 4H, CH₂), 1.23-1.37 (m, 4H, CH₂), 1.68 (s, 3H, =C-CH₃), 1.75-1.88 (m, 2H, CH₂-P), 3.02 (quart., 2H, CH₂-N), 3.15 (t, 2H, CH₂-O), 3.33-3.44 (m, 2H, CH₂-O), 3.82 (quin, 4H, CH₂-O-P) 5.02 (s, 1H, CH=), 5.44 (s, 1H, CH=), 6.64 (t, 1 H, NH).
¹³C-NMR (63 MHz, CDCl₃, ppm) 15.57 and 15.67 (d, POCH₂CH₃), 18.01, 25.04, 26.00 and 27.22 (d, CH₂-P), 28.70 38.77, 60.68 and 60.79 (d, POCH₂CH₃), 63.66 (CH₂-O), 70.00 (CH₂-O), 118.10 (CH₂=), 139.56 (=C-CH₃), 167.82 (C=O).

### N-[9-(dihydroxyphosphoryl)-7-oxa-nonyl] methacrylamide (4)

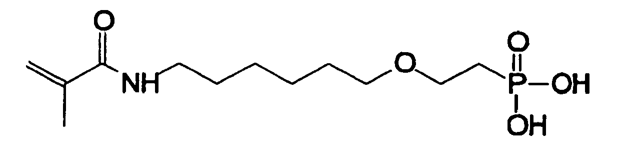

To a solution of 18.57 g (53.1 mmol) of N-[9-(diethoxyphosphoryl)-7-oxa-nonyl] methacrylamide 3 in 100 ml methylene chloride 22.06 g (144.1 mmol) trimethylsilyl bromide were added dropwise at room temperature. The mixture was refluxed for 4 h before the solvent was evaporated at a rotary evaporator and the residue solved again in 200 ml methanol. After stirring this solution at room temperature for 2 h and removal of the solvent a brownish oil was obtained as raw product. The material was solved in 200 ml methylene chloride and extracted once with an aqueous solution of 4.1 g sodium hydroxide in 120 ml water. After the aqueous layer had been separated and washed four times with 100 ml methylene chloride, it was poured over an acidic ion exchange column (Merck ion exchanger I). The resulting acidic aqueous solution was narrowed down at the rotary evaporator and extracted three times with 100 ml methylene chloride. 0.025 mol% BHT were added, before the aqueous layer was concentrated at the rotary evaporator and then dried under high vacuum (3 x 10⁻³mbar) at 40°C until the weight was constant. This afforded 11.24 g (yield: 72%) of a clear, brownish oil.
IR(film, cm⁻¹) 3317 (b,m), 2931 (m), 2862 (m), 1648 (w), 1545 (b, s), 1446 (m), 1373 (m), 1096 (s), 997 (s), 926 (s), 781 (s), 714 (s).
¹H-NMR (250 MHz, d₆-DMSO, ppm) 1.14-1.30 (m, 4H, CH₂), 1.33-1.51 (m, 4H, CH₂), 1.81 (s, 3H, =C-CH₃), 1.75-1.95 (m, 2H, CH₂-P), 3.06 (quart., 2H, CH₂-N), 3.31 (t, 2H, CH₂-O), 3.50 (quart., 2H, CH₂-O). 5.26 (s, 1H, CH=), 5.60 (s, 1H, CH=), 7.91 (t, 1H, NH), 10.04 (broad s, 2H, P-O-H).
¹³C-NMR (63 MHz, d₆-DMSO, ppm) 18.82, 25.51 and 26.39 (d, CH₂-P), 27.67, 29.19, 29.79, 38.88, 65.18 (CH₂-O), 69.87 (CH₂-O), 118.84 (CH₂=), 140.14 (=C-CH₃), 167.45.(C=O).

### Example 3

### N-[2-(diethoxyphosphoryl)-ethyl] acrylamide (5)

To a solution of 8.09 g (44.7 mmol) (2-aminoethyl)phosphonic acid diethyl ester in 150 ml methylene chloride a solution of 6.06 g (67 mmol) acryloyl chloride in 30 ml methylene chloride and a solution of 2.68 g (67 mmol) sodium hydroxide in 30 ml water were added simultaneously under stirring, so that the temperature remains at 0-5 °C. Thereafter the mixture was stirred at room temperature for additional two hours. The reaction was terminated by the addition of 100 ml water. To achieve separation of the layers some sodium chloride was added. The organic phase was separated and the aqueous solution was extracted twice with 50 ml methylene chloride. The combined organic liquids were washed with 50 ml of 1 n HCl, 50 ml of 1 n NaHCO₃ and with 50 ml water. Drying over magnesium sulfate, filtration and evaporation of the solvent yielded a yellow oil as raw product. As final purification the material was chromatographed on a silica gel column with ethyl acetate as eluens (R_{f} = 0.27). This afforded 6.72 g (yield: 63%) of a yellowish oil, which was stabilized by the addition of 0.025 mol% BHT.

IR(film, cm⁻¹) 3274 (m), 2983 (m), 2937 (w), 1660 (m), 1544 (m), 1444 (m), 1310 (m), 1219 (s), 1021 (s), 954 (s), 828 (m), 788 (m), 698 (m).
¹H-NMR (250 MHz, CDCl₃, ppm) 1.14 (t, 6H, CH₃), 1.81-1.94 (m, 2H, CH₂-P), 3.31-3.45 (m, 2H, CH₂-N), 3.85-3.97 (m, 4H, CH₂-O), 5.40-5.44 (dd, 1H, CH=C-CO), 5.93-6.13 (m, 2H, CH=CH-CO), 7.32 (t, 1H, NH).
¹³C-NMR (63 MHz,, CDCl₃ ppm) 15.93 and 16.04 (d, POCH₂CH₃), 23.96 and 26.17 (d, CH₂-P), 33.26 (CH₂-N), 61.39 and 61.50 (d, POCH₂CH₃), 119.08 (C=), 139.30 (C=), 165.41 (C=O).

### N-[2-(dihydroxyphosphoryl)-ethyl] acrylamide (6)

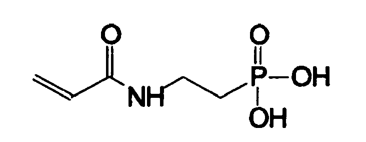

8.86 g (56.32 mmol) trimethylsilyl bromide were added dropwise at room temperature under stirring to a solution of 5.83 g (24.81 mmol) N-[2-(diethoxyphosphoryl)-ethyl] methacrylamide 5 in 30 ml methylene chloride. The reaction mixture was heated under reflux for 4 h. Afterwards the solvent was removed at a rotary evaporator and the residue solved in methanol. This solution was stirred for 16 h at room temperature. Then the solvent was removed and the remaining oil solved in 30 ml water. After the aqueous solution was washed two times with 20 ml methylene chloride, it was stabilized by the addition of 0.025 mol% BHT and concentrated under reduced pressure. After drying of the material under reduced pressure (8 x 10⁻³mbar) at 40 °C for 19 h 4.385 g (yield: 98%) of a highly viscose, yellowish oil were obtained.

IR(film, cm⁻¹) 2812 (b, s), 2359 (m), 1649 (m), 1547 (s), 1444 (m), 1365 (m), 1121 (s), 927 (s), 793 (s), 713 (s).

¹H-NMR (250 MHz, d₆-DMSO, ppm) 1.77-1.99 (m, 2H, CH₂-P), 3.25-3.44 (m, 2H, CH₂-N), 5.62 (dd, 1H, CH=C-CO), 6.07-6.32 (m, 2H, CH=CH-CO), 8.40 (t, 1H, NH).
¹³C-NMR (63 MHz, d₆-DMSO, ppm) 27.11 and 29.23 (d, CH₂-P), 34.05 (CH₂-N), 125.74 (C=), 131.89 (C=), 165.14 (C=O).

### Example 4

### N-[2-(diethoxyphosphoryl)-ethyl]-N-butyl amine (7)

30.04 g (0.411 mol) n-butyl amine and 67.41 g (0.411 mol) diethyl vinylphosphonate were stirred at 65 °C for 24 h. This afforded 97.32 g (yield: 100%) of a colorless oil.
IR(film, cm⁻¹) 3411, 3390 (OH), 2973, 2929, 2885 (CH₂/CH₃), 1390 (CH₂/CH₃), 1078 cm⁻¹ (OH).
¹H-NMR (250 MHz, CDCl₃, ppm) 1.39 (t, 3H, CH₃), 1.79-2.20 (m, 10H, CH₂, OCH₂C*H*₃), 2.40-2.71 (m, 2H, CH₂-P), 3.02-3.29 (m, 2H, CH₂-N), 3.39-3.62 (m, 2H, CH₂-N), 4.57-4.84 (m, 4H, POCH₂).
¹³C-NMR (63 MHz, CDCl₃, ppm) 13.70 (C₃H₆-CH₃), 16.23 (OCH₂-CH₃), 20.26, 25.18 and 27.36 (d, CH₂-P), 31.86, 43.14, 49.08, 61.35 (OCH₂-CH₃).

### N-[2-(diethoxyphosphoryl)-ethyl]-N-butyl acrylamide (8)

To a solution of 55.27 g (0.233 mol) N-[2-(diethoxyphosphoryl)-ethyl]-N-butyl amine 7 in 100 ml methylene chloride a solution of 23.19 g (0.256 mol) acryloyl chloride in 130 ml methylene chloride and a solution of 10.25 g (0.256 mol) sodium hydroxide in 200 ml water were added simultaneously under stirring, so that the temperature remains at 0-5 °C. Thereafter the mixture was stirred at room temperature for additional two hours. The reaction was terminated by the addition of 100 ml water. The organic layer was separated and the aqueous solution was extracted twice with 30 ml methylene chloride. The combined organic liquids were washed with 150 ml of 1 n HCl, 150 ml of 1 n NaHCO₃ and with 150 ml water. The solution of the product was stabilized with 0.025 mol% BHT and dried over sodium sulfate. Filtration and evaporation of the solvent yielded 40.07 g (yield: 59 %) of a colorless oil.

IR(film, cm⁻¹) 2978/2962/2956 (CH₃/CH₂), 1648 (CO), 1614 (C=C), 1456/1431/1371 (CH₃/CH₂), 794 (C=C)
¹H-NMR (250 MHz, CDCl₃, ppm) 0.79 (t, 3H, CH₃), 1.11-1.31 (m, 8H, CH₂, OCH₂C*H*₃), 1.33-1.54 (m, 2H, CH₂), 1.87-2.12 (m, 2H, CH₂-P), 3.12-3.32 (m, 2H, CH₂-N), 3.38-3.57 (m, 2H, CH₂-N), 3.88-4.17 (m, 4H, POCH₂), 5.50-5.67 (m, 1H, CH=C-CO), 6.13-6.30 (m, 1H, CH=C-CO), 6.33-6.52 (m, 1H, C=CH-CO).
¹³C-NMR (63 MHz, CDCl₃, ppm) 13.41 (C₃H₆-CH₃), 16.02 and 16.11 (d, POCH₂-CH₃), 19.57, 22.78 and 24.94 (d, CH₂-P), 31.40, 41.35, 48.19, 61.35 and 61.45 (d, POCH₂-CH₃), 127.16 (C=), 127.62 (C=), 165.72 (C=O).

### N-[2-(dihydroxyphosphoryl)-ethyl]-N-butyl acrylamide (9)

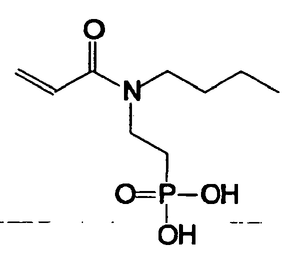

33.56 g (0.219 mol) trimethylsilyl bromide were added dropwise at room temperature under stirring to a solution of 31.93 g (0.11 mol) N-[2-(diethoxyphosphoryl)-ethyl] methacrylamide 8 in 100 ml methylene chloride. The reaction mixture was heated under reflux for 4 h. Afterwards the solvent was removed at a rotary evaporator and the residue solved in 100 ml methanol. This solution was stirred for 4 h at room temperature. The solution of the product was stabilized by the addition of 0.025 mol% BHT and concentrated under reduced pressure. After drying of the material under reduced pressure (8 x 10⁻³mbar) at 40°C for 24 h 21.65 g (yield: 84%) of a white solid were obtained.

IR(ATR, cm⁻¹) 3411, 3390 (OH), 2973, 2929, 2885 (CH₂/CH₃), 1390 (CH₂/CH₃), 1078 cm⁻¹ (OH).
¹H-NMR (250 MHz, d₆-DMSO, ppm) 0.97 (t, 3H, CH₃), 1.27-1.45 (m, 2H, CH₂), 1.47-1.66 (m, 2H, CH₂), 1.93-2.17 (m, 2H, CH₂-P), 3.35-3.52 (m, 2H, CH₂-N), 3.55-3.75 (m, 2H, CH₂-N), 5.69-5.83 (m, 1H, CH=C-CO), 6.24 (dd, 1H, CH=C-CO), 6.64-6.81 (m, 1H, C=CH-CO).
¹³C-NMR (63 MHz, d₆-DMSO, ppm) 14.2 (C₃H₆-CH₃), 21.2, 25.8 and 27.9 (d, CH₂-P), 30.8, 32.6, 43.8, 128.9 (C=), 129.1 (C=), 168.4 (C=O).

### Example 5

### (2,2 (4),4)-Trimethylhexamethylene bis(acrylamide) (10)

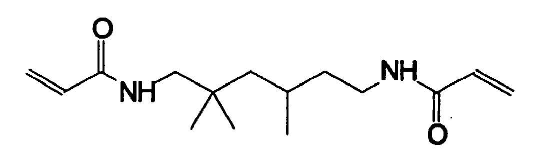

To a solution of 60 g (0.379 mol) (2,2 (4),4)-trimethylhexamethylene diamine in 100 ml methylene chloride a solution of 72.05 g (0.796 mol) acryloyl chloride in 130 ml methylene chloride and a solution of 31.84 g (0.796 mol) sodium hydroxide in 200 ml water were added simultaneously under stirring, so that the temperature remains at 0-5 °C. Thereafter the mixture was stirred at room temperature for additional two hours. The reaction was terminated by the addition of 100 ml water. The organic phase was separated and the aqueous solution was extracted twice with 30 ml methylene chloride. The combined organic liquids were washed with 150 ml of 1 n HCl, 150 ml of 1 n NaHCO₃ and with 150 ml water. The solution of the product was stabilized with 0.025 mol% BHT and dried over sodium sulfate. Filtration and evaporation of the solvent yielded 87 g (yield: 86 %) of a colorless, highly viscose oil.

IR(film cm⁻¹) 3411, 3278 (NH), 2957 (CH2/CH3), 1656 (NHCO), 1546 (C=C), 1241 (CH2/CH3), 984/956 (C=C), 703 (C=C).
¹H-NMR (250 MHz, CDCl₃, ppm) 0.7 - 3.4 (several m, 18H, CH₃, CH₂), 5.51-5.60 (m, 1H, CH=C-CO), 6.16-6.26 (m, 2H, CH=CH-CO), 7.12, 6.84, 6.70, 6.59 (4 x t, 2H, NH).
¹³C-NMR (63 MHz, CDCl₃, ppm) 20.80, 22.21, 25.36, 26.01, 26.75, 27.90, 28.02, 29.04, 30.73, 32.80, 35.00, 35.68, 37.21, 38.79, 40.51, 45.16, 46.02, 47.02, 48.70, 125.66 (C=), 131.08 (C=), 165.82 (C=O), 166.09 (C=O).

### Example 6

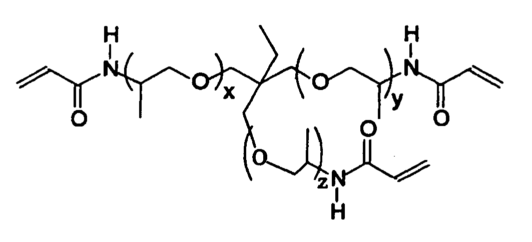

### JEFFAMINE T-403 polyoxypropylenetriamine tris(acrylamide) (11)

To a solution of 73.25 g (0.166 mol) JEFFAMINE T-403 polyoxypropylenetriamine (x +y+z = 5.3) in 100 ml methylene chloride a solution of 47.46 g (0.524 mol) acryloyl chloride in 130 ml methylene chloride and a solution of 20.98 g (0.524 mol) sodium hydroxide in 200 ml water were added simultaneously under stirring, so that the temperature remains at 0-5 °C. Thereafter the mixture was stirred at room temperature for additional two hours. The reaction was terminated by the addition of 300 ml water. The organic phase was separated and the aqueous solution was extracted twice with 100 ml methylene chloride. The combined organic liquids were washed with 150 ml of 1 n HCl, 150 ml of 1 n NaHCO₃ and with 150 ml water. The solution of the product was stabilized with 0.025 mol% BHT and dried over sodium sulfate. Filtration and evaporation of the solvent yielded 86.1 g (yield: 85 %) of a clear, yellowish, highly viscose oil.

IR(film cm⁻¹) 3411, 3280 (OH), 2970, 2929, 2872 (CH₂/CH₃), 1657/1622 (CONH), 1542 (C=C), 1406/1374 (CH₂/CH₃), 1101 cm⁻¹ (ROR).
¹H-NMR (250 MHz, CDCl₃, ppm) 0.74-0.95 (t), 0.99-1.25 (m), 1.27-1.53 (m), 2.90-3.73 (m), 4.04-4.31 (broad s), 5.49-5.73 (m, 1 H, CH=C-CO), 5.90-6.43 (m, 2H, CH=CH-CO).
¹³C-NMR (63 MHz, CDCl₃, ppm) 7.5 (CH₂-*C*H₃), 17.0/17.5 (CH-*C*H₃), 22.9, 32.0, 45.1/45.2 (C-N), 71.6/74.8/75.1 (CH₂-O), 125.7 (C=), 131.1 (C=), 164.9 (C=O).

### Test 1

### Examination of the hydrolytic stability of N-substituted alkylacrylic or acrylic acid amide monomers, which contain an acidic moiety

As a typical example of a N-substituted alkylacrylic or acrylic acid amide monomer N-[2-(dihydroxyphosphoryl)-ethyl]-N-butyl acrylamide 9 and as a comparative example dihydroxyphosphorylmethyl methacrylester were subjected to the following conditions:

1.5 mmol of the acrylic acid amide monomer was solved in 5g of a mixture consisting of 50 wt% ethanol and 50 wt% water. The solutions were stored a in closed vial at 50 °C. The kind and the extend of hydrolysis was determined by HPLC.
After 1 week 50 % and after 6 weeks 83 % of the ester is hydrolyzed into methacrylic acid and hydroxymethylphosphonic acid, whereas the acrylic amide 9 does show a hydrolysis into acrylic acid and amine of 0 % after 1 week and 7.9 % after 6 weeks.

### Test 2

### Examination of the hydrolytic stability of N-substituted acrylic acid amide monomers which do not contain an acidic moiety

As a model compound for a N-substituted acrylic acid amide monomer n-butyl acrylamide and as a comparative example n-butyl acrylester were subjected to the following conditions:
1.5 mmol of the acrylamide monomer was solved together with 1.5 mmol ethanesulfonic acid in 5g of a mixture consisting of 50 wt% ethanol and 50 wt% water.
The solutions were stored in closed vial at 50 °C. The kind and the extend of hydrolysis was determined by HPLC.
After 1 week 62 % of the ester is hydrolyzed into acrylic acid and butanol, whereas the acrylamide shows no hydrolysis until 7 weeks.

### Application Example 1

281.85 mg (2,2 (4),4)-Trimethylhexamethylene bis(acrylamide) (10), 416,85 mg JEFFAMINE T-403 polyoxypropylenetriamine tris(acrylamide) (11), 286.00 mg N-[2-(dihydroxyphosphoryl)-ethyl]-N-butyl acrylamide (9), 17.70 mg bis(2,4,6-trimethylbenzol)-phenylphosphine oxide, 8.20 mg dimethylaminoethyl benzoic acid ethylester and 7.10 mg camphor quinone were dissolved in solution consisting of 166.67 mg formic acid, 66.67 mg ethanol and 266.67 mg water.

### Preparation of the teeth

For the adhesion the enamel is abraded with 500 grit silicon carbide paper so that an approximately flat area of enamel about 5 mm in diameter is present. The teeth are then washed under running water and used within 2 hours as below.

### Preparation of the adhesion samples

Gelatine capsules (#5 supplied by Torpac Inc.) for the tests are filled to about two thirds of their length with Spectrum TPH and this is hardened by placing the capsules in a light oven. Six teeth were prepared for the test.
The tooth surface to be adhered to is dried lightly with a paper tissue or a 5 second blast of air, and the treatment solution is applied using an applicator tip or brush. The material is left in contact with the tooth for twenty seconds, dried with air for 5 seconds and light cured for 10 second with Spectrum lamp 800. Spectrum TPH is then filled into the remaining space of the pre-filled gelatine capsule and it is placed on to the prepared enamel surface. Spectrum TPH is then cured by irradiating three times for twenty seconds at equally spaced intervals around the capsule.

After storage at 37 °C for 2 hours the adhesion to enamel is 10.5 ± 2.8 MPa.

If the samples prepared according to the above described procedure and thermocycled 1800 times between 5°C and 55°C with a dwell time in each bath of 20 seconds an adhesion to enamel of 10.7 ± 2.2 MPa was measured.

### Further Examples:

### Hydrolysis stable polymerizable N-substituted alkylacrylic and acrylic acid amide monomers

### Example 7

### N,N'-bismethacryloyl-N,N'-dibenzyl-5-oxanonanediamine-1.9

In a 4-necked 1-I-flask equipped with a stirrer, a thermometer and two 50 ml dropping funnels 102.16 g (0.3 mol) of N,N'-dibenzyl-5-oxanonanediamine-1.9 were dissolved in 300 ml of methylenechloride. After cooling to 0-5 °C 65.854 g (0.63 mol) of methacryloyl chloride dissolved in 30 ml of methylenechloride and 25.20 g (0.63 mol) of NaOH dissolved in 60 ml of water were added simultaneously under stirring during 1.5 hours so that the temperature remains at 0-5 °C. Thereafter the mixture were stirred at room temperature for additional two hours. Than the reaction mixture were hydrolyzed with 600 ml of ice-water. The organic phase were separated and the aqueous solution were extracted twice with methylenechloride. The collected organic liquids were washed with 150 ml of 1 n HCl, 150 ml of 1 n NaHCO₃ and sometimes with 150 ml of deionised water until the water shows a pH-value of approximately 7. Than the organic solution was dried over NaSO₄. Thereafter the NaSO₄ was filtered off and to the solution 0.1346 g of 2,6-di-tert.-butyl-p-cresol were added. The methylenechloride was removed at 40 °C in vacuum and the bismethacrylamide was dried.
Yield: 136.66 g (95.6 % of th.), n_{D}²⁰ = 1.5383, η = 1.65 Pa*s
C₃₀H₄₀N₂O₃, 476.65
IR: 2941 (CH₂/CH₃), 3086/3062/3030, (Ar), 1647 (CONR), 1626 (CH₂=CH-), 1119 cm⁻¹ (ROR)

### Example 8

### N,N'-bisacryloyl-N,N'-dibenzylethylenediamine

In a 4-necked 1-I-flask equipped with a stirrer, a thermometer and two 50 ml dropping funnels 29.198 g (0.12 mol) of N,N'-dibenzylethylenediamine were dissolved in 100 ml of methylenechloride. After cooling to 0-5 °C 21.991 g (0.24 mol) of acryloylchloride dissolved in 30 ml of methylenechloride and 9.718 g (0.24 mol) of NaOH dissolved in 40 ml of water were added simultaneously under stirring during 1.5 hours so that the temperature remains at 0-5 °C. Thereafter the mixture were stirred at room temperature for additional two hours. Than the reaction mixture were hydrolyzed with 600 ml of ice-water. The organic phase were separated and the aqueous solution were extracted twice with methylenechloride. The collected organic liquids were washed with 100 ml of 1 n HCl, 100 ml of 1 n NaHCO₃ and sometimes with 100 ml of deionised water until the water shows a pH-value of approximately 7. Than the organic solution was dried over NaSO₄. Thereafter the NaSO₄ was filtered off and to the solution 0.028 g of 2,6-di-tert.-butyl-p-cresol were added. The methylenechloride was removed at 40 °C in vacuum and the bismethacrylamide was dried.
Yield: 27.9 g (65.9 % of th.), mₚ = 75,5-76,6 °C, Tg = -7.2 °C, Mₙ (vpo) = 350g/mol

| | | | | |
|---|---|---|---|---|
| C₂₂H₂₄N₂O₂, 348.45 | calc. | C 75.83 | H 6.94 | N 8.04 |
| | found | C 76.00 | H 7.26 | N 8.05 |

### Example 9

### N,N'-bisacryloyl-N,N'-dibenzyl-4,4'-diaminodicyclohexylamine

In a 4-necked 1-I-flask equipped with a stirrer, a thermometer and two 50 ml dropping funnels 60.551 g (0.16 mol) of N,N'-dibenzyl-4,4'-diaminodicyclohexylamine were dissolved in 150 ml of methylenechloride. After cooling to 0-5 °C 28.061 g (0.31 mol) of acryloylchloride dissolved in 30 ml of methylenechloride and 12.401 g (0.31 mol) of NaOH dissolved in 50 ml of water were added simultaneously under stirring during 1.5 hours so that the temperature remains at 0-5 °C. Thereafter the mixture were stirred at room temperature for additional two hours. Than the reaction mixture were hydrolyzed with 500 ml of ice-water. The organic phase were separated and the aqueous solution were extracted twice with methylenechloride. The collected organic liquids were washed with 100 ml of 1 n HCl, 100 ml of 1 n NaHCO₃ and sometimes with 10 ml of deionised water until the water shows a ph-value of approximately 7. Than the organic solution was dried over NaSO₄. Thereafter the NaSO₄ was filtered off and to the solution 0.077 g of 2,6-di-tert.-butyl-p-cresol were added. The methylenechloride was removed at 40 °C in vacuum and the bismethacrylamide was dried.
Yield: 54.0 g (69.9 % of th.), Tg = 47.1 °C

### Example 10

### 3,(4),8,(9)-Bis(2-propene amido methyl)-tricyclo-5.2.1.0 ^{2,6} decane

In a 4-necked 1-I-flask equipped with a stirrer, a thermometer and two 50 ml dropping funnels 96.01 g (0.35 mol) of 3,(4),8,(9)-Bis(aminomethyl)-tricyclo-5.2.1.0^{2,6} decane were dissolved in 350 ml of methylenechloride. After cooling to 0-5 °C 76.54 g (0.735 mol) of methacryloyl chloride dissolved in 35 ml of methylenechloride and 29.40 g (0.735 mol) of NaOH dissolved in 70 ml of water were added simultaneously under stirring during 1.5 hours so that the temperature remains at 0-5 °C. Thereafter the mixture were stirred at room temperature for additional two hours. Than the reaction mixture were hydrolyzed with 600 ml of ice-water. The organic phase were separated and the aqueous solution were extracted twice with methylenechloride. The collected organic liquids were washed with 150 ml of 1 n HCl, 150 ml of 1 n NaHCO₃ and sometimes with 150 ml of deionised water until the water shows a pH-value of approximately 7. Than the organic solution was dried over NaSO₄. Thereafter the NaSO₄ was filtered off and to the solution 0.1157 g of 2,6-di-tert.-butyl-p-cresol were added. The methylenechloride was removed at 40 °C in vacuum and the bismethacrylamide was dried.
Yield: 106.02 g (91.6 % of th.)
C₂₀H₃₀N₂O₂, 330.47
IR: 2941 (CH₂/CH₃), 3330/1647 (CONHR), 1626 (CH₂=CH-)

### Example 11

### N,N'-Bismethacryloyl-3.6-dioxaoctanediamine-1.8

In a 4-necked 1-I-flask equipped with a stirrer, a thermometer and two 50 ml dropping funnels 59.28 g (0.4 mol) of 3.6-dioxaoctanediamine-1.8 were dissolved in 300 ml of methylenechloride. After cooling to 0-5 °C 87.47 g (0.84 mol) of methacryloyl chloride dissolved in 40 ml of methylenechloride and 33.60 g (0.84 mol) of NaOH dissolved in 80 ml of water were added simultaneously under stirring during 1.5 hours so that the temperature remains at 0-5 °C. Thereafter the mixture were stirred at room temperature for additional two hours. Than the reaction mixture were hydrolyzed with 600 ml of ice-water. The organic phase were separated and the aqueous solution were extracted twice with methylenechloride. The collected organic liquids were washed with 150 ml of 1 n HCl, 150 ml of 1 n NaHCO₃ and sometimes with 150 ml of deionised water until the water shows a pH-value of approximately 7. Than the organic solution was dried over NaSO₄. Thereafter the NaSO₄ was filtered off and to the solution 0.1137 g of 2,6-di-tert.-butyl-p-cresol were added. The methylenechloride was removed at 40 °C in vacuum and the bismethacrylamide was dried.
Yield: 95.20 g (83.7 % of th.), n_{D}²⁰ = 1.5042. η = 2.17 Pa*s
C₁₄H₂₄N₂O₄, 284.35
IR: 2926/2870 (CH₂/CH₃), 3336/1659 (CONHR), 1620 (CH₂=CH-), 1130 cm⁻¹ (ROR)

## Claims

1. An aqueous one-pack self-etching and self-priming dental adhesive composition having a pH of at most 2, which comprises:
(i) a polymerizable N-substituted alkylacrylic or acrylic acid amide monomer which optionally contains an inorganic acidic moiety selected from a phosphonic acid moiety or a sulfonic acid moiety,
(ii) a curing system, and
(iii) a polymerizable N-substituted alkylacrylic or acrylic acid amide monomer containing at least an inorganic acidic moiety selected from a phosphonic acid moiety or a sulfonic acid moiety,
wherein
the polymerizable N-substituted alkylacrylic or acrylic acid amide monomer (i) or
the polymerizable N-substituted alkylacrylic or acrylic acid amide monomer containing at least an inorganic acidic moiety (iii) contains at least two polymerizable groups.

2. The composition of claim 1, which comprises an organic or inorganic acid.

3. The composition of claim 2, whereby the organic acid is selected from the group consisting of methacrylic acid, acrylic acid, fumaric acid, maleic acid, citric acid, itaconic acid, and formic acid and whereby the inorganic acid is selected from phosphoric acid, sulfuric acid and hydrofluoric acid.

4. The composition of any of the preceding claims, which contains beside water an organic water-soluble solvent.

5. The composition of claim 4, whereby the organic water-soluble solvent is selected from ethanol, propanol, butanol, acetone, and methyl ethyl ketone.

6. The composition of any of the preceding claims, wherein the at least two polymerizable groups are directly or indirectly linked via an amide bond.

7. The composition of any of the preceding claims, which further contains a nanofiller.

8. The composition of any of the preceding claims wherein the polymerizable N-substituted alkylacrylic or acrylic acid amide monomer is selected from the group consisting of compounds represented by the following formulas: 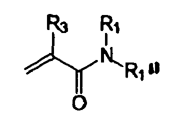 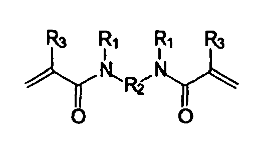 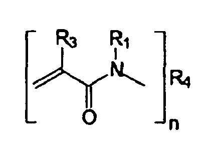 wherein
R₁, R_{1''} and R₃ independently are hydrogen or a substituted or unsubstituted C₁ to C₁₈ alkyl group, a substituted or unsubstituted cycloalkyl group, a substituted or unsubstituted C₅ to C₁₈ aryl or heteroaryl group, a substituted or unsubstituted C₅ to C₁₈ alkylaryl or alkylheteroaryl group, a substituted or unsubstituted C₇ to C₃₀ aralkyl group,
provided that in case one of R₁ and R_{1''} is hydrogen in formula (F1) the other is not hydrogen;
R₂ is a difunctional substituted or unsubstituted C₁ to C₁₈ alkylene, difunctional substituted or unsubstituted cycloalkylene, a difunctional substituted or unsubstituted C₅ to C₁₈ aryl or heteroaryl group, a difunctional substituted or unsubstituted C₅ to C₁₈ alkylaryl or alkylheteroaryl group, a difunctional substituted or unsubstituted C₇ to C₃₀ aralkyl group,
R₄ is a mono- or polyfunctional substituted or unsubstituted C₁ to C₁₈ carbon chain group, a mono- or polyfunctional substituted or unsubstituted cycloalkyl group, a mono- or polyfunctional substituted or unsubstituted C₅ to C₁₈ aryl or heteroaryl group, a mono- or polyfunctional substituted or unsubstituted C₅ to C₁₈ alkylaryl or alkylheteroaryl group, a mono- or polyfunctional substituted or unsubstituted C₇ to C₃₀ aralkyl group, and
n is an integer.

9. The composition according to claim 8, **characterized in that** R₂ and R₄ independently is a di- or polyfunctional substituted or unsubstituted C₁ to C₁₈ carbon chain group or a di- or polyfunctional substituted or unsubstituted cycloalkylene group, which has at least one linkage of ether, thioether, ester, thiocarbonyl, amide, carbonyl, sulfonyl, urethane, or substituted or unsubstituted amine linkages.

10. The composition according to claim 9, **characterized in that**
R₂ or R₄ is -(CH₂)₂-O-(CH₂)₂-O-(CH₂)₂-, 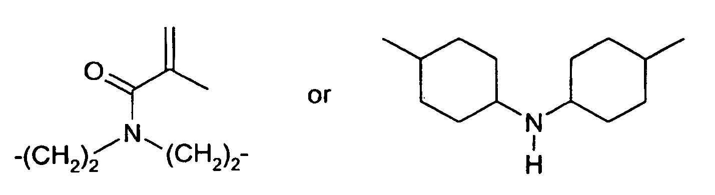 and
n is 2.

11. The composition according to one of the preceding claims, **characterized in that** the polymerizable N-substituted alkylacrylic or acrylic acid amide monomer which optionally contains an inorganic acidic moiety selected from a phosphonic acid moiety or a sulfonic acid moiety is selected from the group consisting of compounds represented by the following formulas: 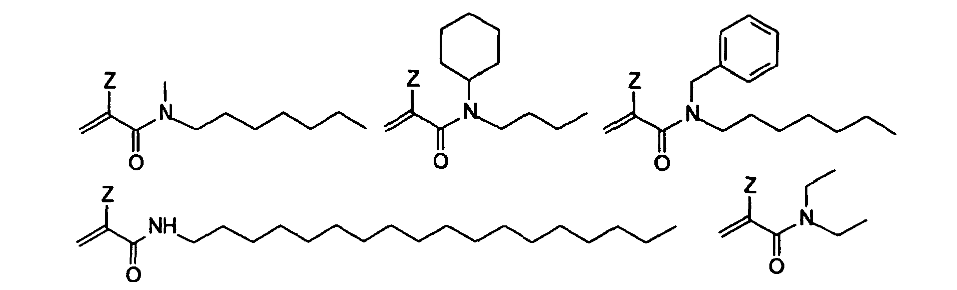 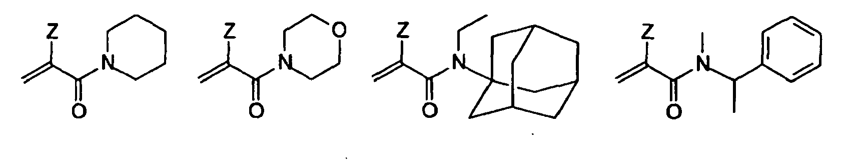 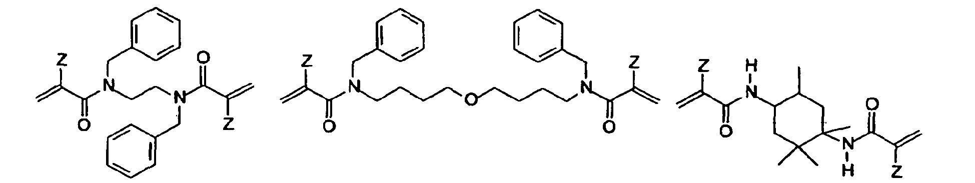 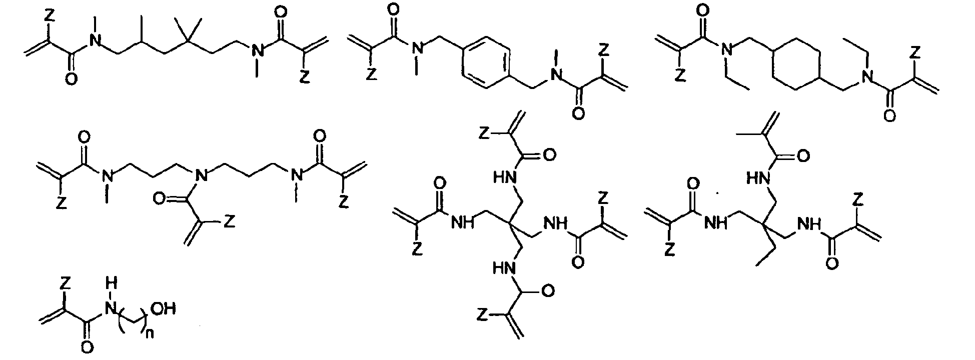 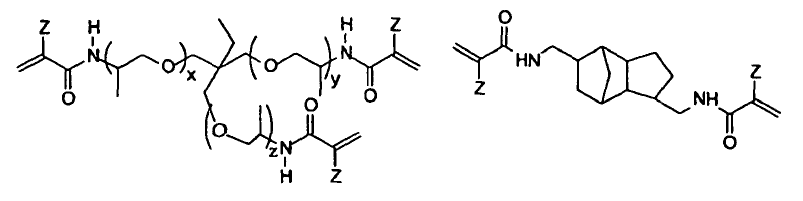 wherein
n is an integer of from 2 to 6;
x, y, and z independently is an integer of from 1 to 10; and
Z is H or a substituted or unsubstituted C₁ to C₁₈ alkyl group.

12. The composition according to one of the preceding claims, **characterized in that** the polymerizable N-substituted alkylacrylic or acrylic acid amide monomer which optionally contains an inorganic acidic moiety selected from a phosphonic acid moiety or a sulfonic acid moiety is selected from (meth)acrylamide monomers.

13. The composition according to one of the preceding claims, **characterized in that** the polymerizable N-substituted alkylacrylic or acrylic acid amide monomer containing at least an inorganic acidic moiety is selected from the group consisting of compounds represented by the following formulas: 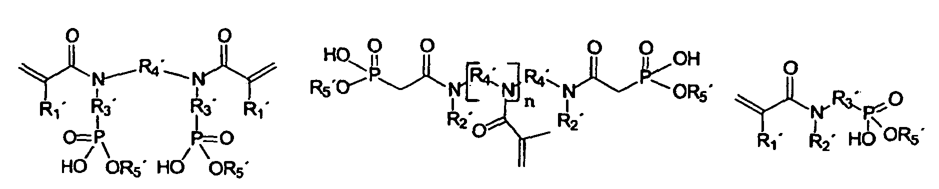 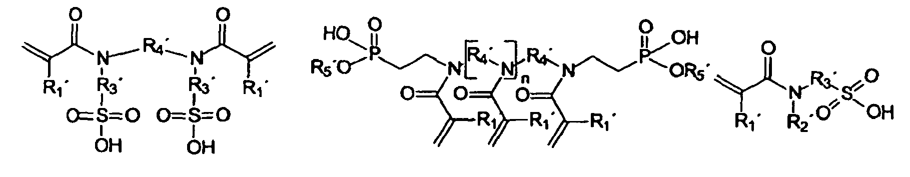 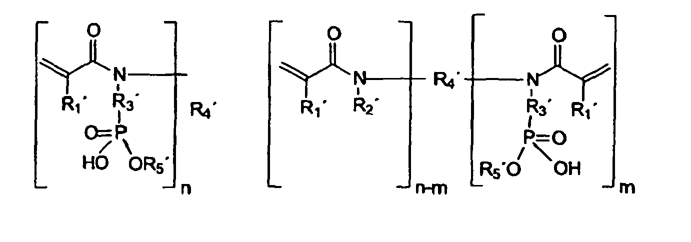 wherein
R_{1'} and R_{2'} represent independently from each other a hydrogen atom or a substituted or unsubstituted C₁ to C₁₈ alkyl group, a substituted or unsubstituted cycloalkyl group, a substituted or unsubstituted C₅ to C₁₈ aryl or heteroaryl group, a substituted or unsubstituted C₅ to C₁₈ alkylaryl or alkylheteroaryl group, a substituted or unsubstituted C₇ to C₃₀ aralkyl group,
R_{3'} and R_{4'} represent independently from each other a difunctional substituted or unsubstituted C₁ to C₁₈ carbon chain group, a difunctional substituted or unsubstituted cycloalkylene, a difunctional substituted or unsubstituted C₅ to C₁₈ aryl or heteroaryl group, a difunctional substituted or unsubstituted C₅ to C₁₈ alkylaryl or alkylheteroaryl group, a difunctional substituted or unsubstituted C₇ to C₃₀ aralkyl group,
R_{5'} represents H or a substituted or unsubstituted C₁ to C₁₈ alkyl group
n is an integer and
m is an integer 1 to 3.

14. The composition according to claim 13, **characterized in that** R₃. and R₄. independently from each other is a difunctional substituted or unsubstituted C₁ to C₁₈ carbon chain group or a difunctional substituted or unsubstituted cycloalkylene, which has at least one linkage of ether, thioether, ester, thiocarbonyl, amide, carbonyl, sulfonyl, urethane, or substituted or unsubstituted amine linkages.

15. The composition according to claim 14, **characterized in that**
R_{3'} is -(CH₂)₂- or 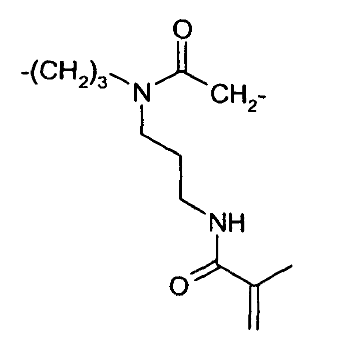 and R_{4'} is -(CH₂)₂-O-(CH₂)₂-O-(CH₂)₂-.

16. The composition according to one of claims 1 to 15, **characterized in that** the polymerizable N-substituted alkylacrylic or acrylic acid amide monomer containing at least an inorganic acidic moiety is selected from one of the monomers according to the following formulas:

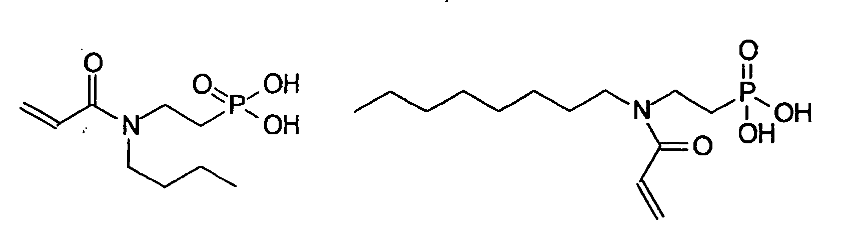

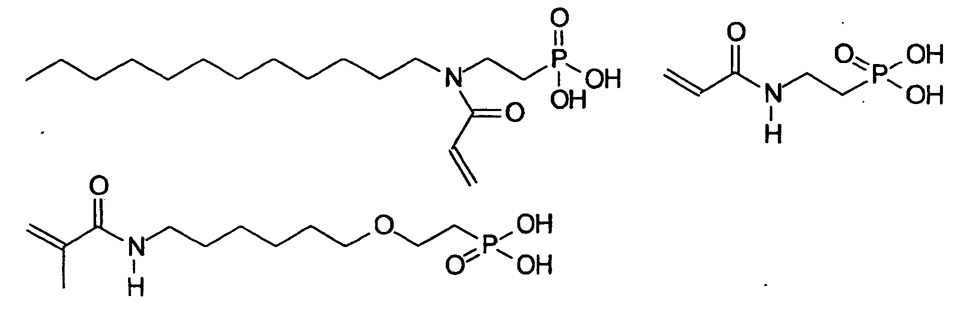

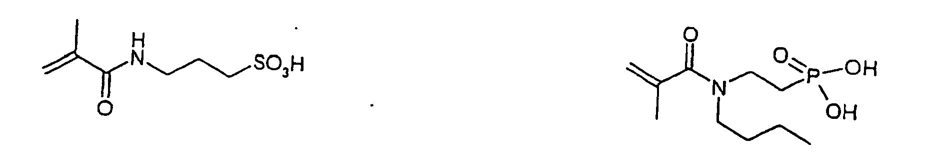

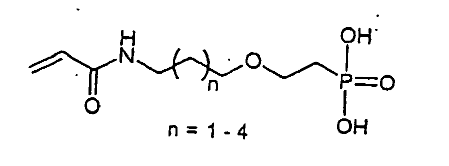

17. The composition according to one of the preceding claims, **characterized in that** the polymerizable N-substituted alkylacrylic or acrylic acid amide monomer containing at least an inorganic acidic moiety is selected from (meth)acrylamide monomers.

18. The composition according to one of the preceding claims, **characterized in that** the curing system comprises a polymerization initiator, an inhibitor or stabilizer.

19. The composition according to any one of the preceding claims, comprising:
(a) a polymerizable N-substituted alkylacrylic or acrylic acid amide monomer having at least two polymerizable moieties,
(b) a polymerizable N-substituted alkylacrylic or acrylic acid amide monomer containing at least an inorganic acidic moiety, and
(c) a curing system.

20. The composition according to claim 19, wherein the polymerizable N-substituted alkylacrylic or acrylic acid amide monomer having at least two polymerizable moieties is selected from the group consisting of compounds represented by the following formulas: 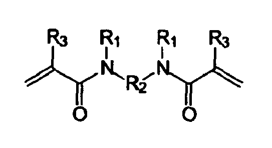 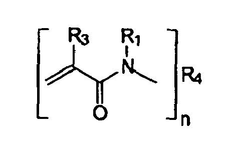 wherein
R₁, R_{1'}, R₂, R₃ and R₄ and n are as defined in claims 8 to 10.

21. The composition according to claim 19 or 20, **characterized in that** the polymerizable N-substituted alkylacrylic or acrylic acid amide monomer is selected from the group consisting of compounds represented by the following formulas: 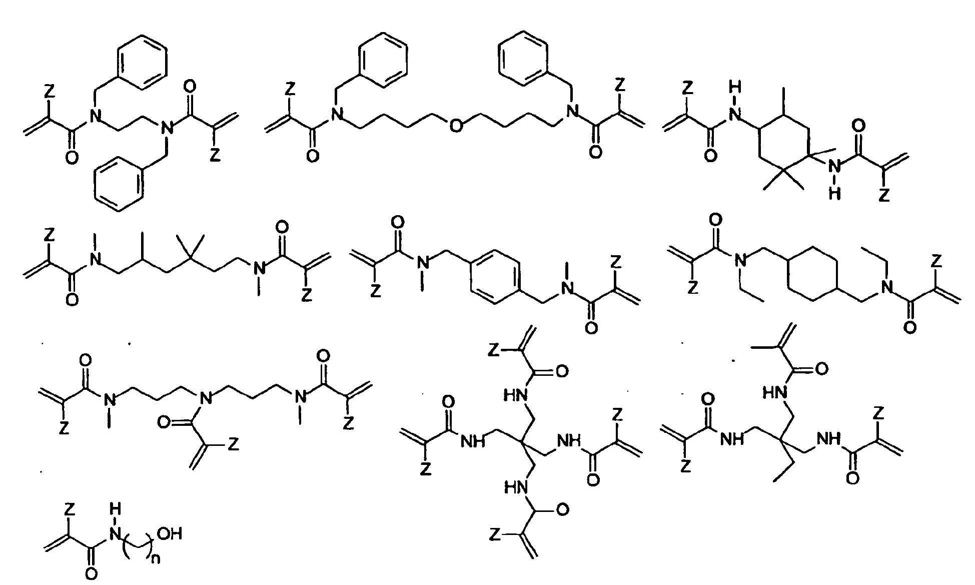 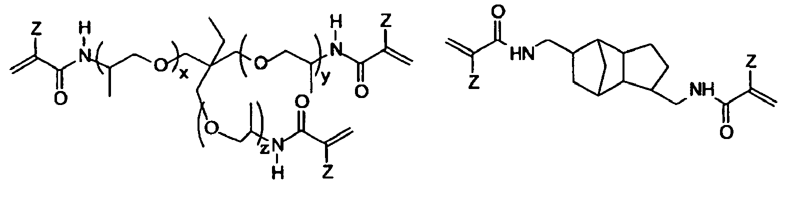
wherein Z is H or a substituted or unsubstituted C₁ to C₁₈ alkyl group and
n, x, y, z are as defined in claim 11.

22. The composition according to one of claims 18 to 20, **characterized in that** the polymerizable N-substituted alkylacrylic or acrylic acid amide monomer is selected from (meth)acrylamide monomers.

23. A kit comprising a container shielded against light, which contains the composition according to one of the preceding claims 1 to 22.

24. The kit according to claim 23, which further comprises instructions for use.

25. A method for the preparation of an aqueous one-pack self-etching and self-priming dental adhesive composition according to any one of the preceding claims, **characterized by** mixing
(A) a polymerizable N-substituted alkylacrylic or acrylic acid amide monomer which optionally contains an inorganic acidic moiety selected from a phosphonic acid moiety or a sulfonic acid moiety,
(B) a curing system,
(C) a water containing solvent, and
(D) a polymerizable N-substituted alkylacrylic or acrylic acid amide monomer containing at least an inorganic acidic moiety selected from a phosphonic acid moiety or a sulfonic acid moiety.

26. A polymerizable N-substituted alkylacrylic or acrylic acid amide monomer containing at least one inorganic acidic moiety selected from a phosphonic acid moiety or sulfonic acid moiety, which is selected from the group consisting of compounds represented by the following formulas: 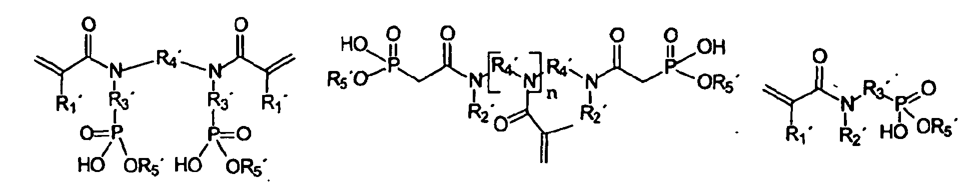 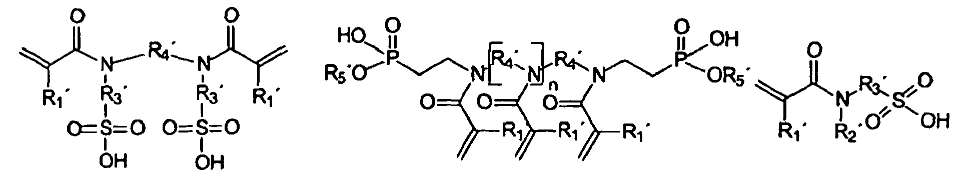 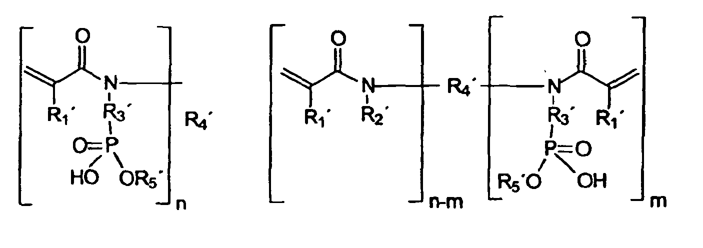 wherein
R_{1'}, R_{2'}, R_{3'}, R_{4'}, R_{5'}, n and m are as defined in claims 13 to 15.

27. Method for the preparation of the polymerizable N-substituted alkylacrylic or acrylic acid amide monomer having at least an inorganic acidic moiety according to claim 25, which comprises
(1) reacting a di- or poly amine compound with a vinylphosphonate or a vinyl sulphonic acid salt;
(2) reacting the product obtained in (1) with an alkylacryloyl or acryloyl halide of the formula CH₂=CR_{1'}-CO-Hal, wherein R_{1'} is as defined in claim 13 and Hal is a halide;
(3) optionally reacting the product obtained in (2) with a trialkyl halogensilane in case of reacting a vinyl phosphonate in step (1); and
(4) hydrolyzing the product obtained in step (2) or (3).

## Patentansprüche

1. Wässerige selbstätzende und selbstgrundierende Dental-Einkomponenten-Kleberzusammensetzung, die einen pH von höchstens 2 aufweist, umfassend:
(i) ein polymerisierbares N-substituiertes Alkylacryl- oder Acrylsäureamidmonomer, das gegebenenfalls eine anorganische Säurekomponente enthält, ausgewählt aus einer Phosphonsäurekomponente oder einer Sulfonsäurekomponente,
(ii) ein Härtungssystem, und
(iii) ein polymerisierbares N-substituiertes Alkylacryl- oder Acrylsäureamidmonomer, das mindestens eine anorganische Säurekomponente enthält, ausgewählt aus einer Phosphonsäurekomponente oder einer Sulfonsäurekomponente,
worin
das polymerisierbare N-substituierte Alkylacryl- oder Acrylsäureamidmonomer (i) oder
das polymerisierbare N-substituierte Alkylacryl- oder Acrylsäureamidmonomer , das mindestens eine anorganische Säurekomponente enthält (iii), mindestens zwei polymerisierbare Gruppen enthält.

2. Zusammensetzung nach Anspruch 1, die eine organische oder anorganische Säure umfasst.

3. Zusammensetzung nach Anspruch 2, worin die organische Säure ausgewählt ist aus der Gruppe bestehend aus Methacrylsäure, Acrylsäure, Fumarsäure, Maleinsäure, Citronensäure, Itaconsäure und Ameisensäure, und worin die anorganische Säure ausgewählt ist aus Phosphorsäure, Schwefelsäure und Fluorwasserstoffsäure.

4. Zusammensetzung nach einem der vorhergehenden Ansprüche, die neben Wasser ein in Wasser lösliches organisches Lösungsmittel enthält.

5. Zusammensetzung nach Anspruch 4, worin das organische wasserlösliche Lösungsmittel ausgewählt ist aus Ethanol, Propanol, Butanol, Aceton und Methylethylketon.

6. Zusammensetzung nach einem der vorhergehenden Ansprüche, worin die mindestens zwei polymerisierbaren Gruppen direkt oder indirekt über eine Amidbindung gebunden sind.

7. Zusammensetzung nach einem der vorhergehenden Ansprüche, die außerdem einen Nanofüllstoff enthält.

8. Zusammensetzung nach einem der vorhergehenden Ansprüche, worin das polymerisierbare N-substituierte Alkylacryl- oder Acrylsäureamidmonomer ausgewählt ist aus der Gruppe bestehend aus durch die folgenden Formeln repräsentierten Verbindungen: 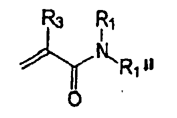 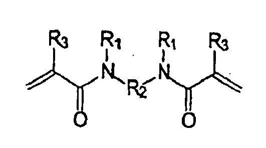 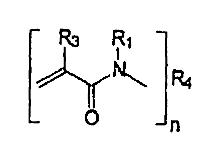 worin bedeuten
R₁, R_{1''} und R₃ unabhängig von einander Wasserstoff oder eine substituierte oder unsubstituierte C₁-C₁₈-Alkylgruppe, eine substituierte oder unsubstituierte Cycloalkylgruppe, eine substituierte oder unsubstituierte C₅-C₁₈-Aryl- oder -Heteroarylgruppe, eine substituierte oder unsubstituierte C₅-C₁₈-Alkylaryl- oder-Alkylheteroarylgruppe, eine substituierte oder unsubstituierte C₇-C₃₀-Aralkylgruppe,
mit der Maßgabe, dass, wenn einer der Reste R₁ und R_{1''} in Formel (F1) Wasserstoff ist, der andere nicht Wasserstoff ist;
R₂ ein difunktionelles substituiertes oder unsubstituiertes C₁-C₁₈-Alkylen, difunktionelles substituiertes oder unsubstituiertes Cycloalkylen, eine difunktionelle substituierte oder unsubstituierte C₅-C₁₈-Aryl- oder -Heteroarylgruppe, eine difunktionelle substituierte oder unsubstituierte C₅-C₁₈-Alkylaryl- oder Alkylheteroarylgruppe, eine difunktionelle substituierte oder unsubstituierte C₇-C₃₀-Aralkylgruppe,
R₄ eine mono- oder polyfunktionelle substituierte oder unsubstituierte C₁-C₁₈-Kohlenstoffkettengruppe, eine eine mono- oder polyfunktionelle substituierte oder unsubstituierte Cycloalkylgruppe, eine mono- oder polyfunktionelle substituierte oder unsubstituierte C₅-C₁₈-Aryl- oder -Heteroarylgruppe, eine mono- oder polyfunktionelle substituierte oder unsubstituierte C₅-C₁₈-Alkylaryl- oder Alkylheteroarylgruppe, eine mono- oder polyfunktionelle substituierte oder unsubstituierte C₇-C₃₀-Aralkylgruppe, und
n eine ganze Zahl.

9. Zusammensetzung nach Anspruch 8, **dadurch gekennzeichnet, dass** R₂ und R₄ unabhängig von einander eine di- oder polyfunktionelle substituierte oder unsubstituierte C₁-C₁₈-Kohlenstoffkettengruppe oder eine di- oder polyfunktionelle substituierte oder unsubstituierte Cycloalkylengruppe sind, die mindestens eine Ether-, Thioether-, Ester-, Thiocarbonyl-, Amid-, Carbonyl-, Sulfonyl-, Urethanbindung oder substituierte oder unsubstituierte Aminbindungen aufweist.

10. Zusammensetzung nach Anspruch 9, **dadurch gekennzeichnet, dass** R₂ oder R₄ ist -(CH₂)₂-O-(CH₂)₂-O-(CH₂)₂-. 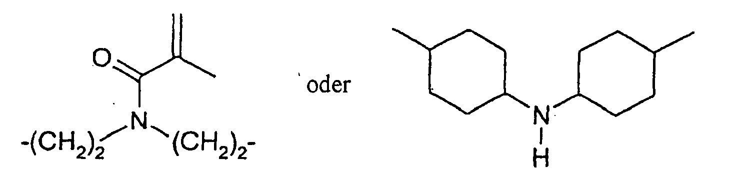 und
n 2 ist.

11. Zusammensetzung nach einem der vorhergehenden Ansprüche, **dadurch gekennzeichnet, dass** das polymerisierbare N-substituierte Alkylacryl- oder Acrylsäureamidmonomer, das gegebenenfalls eine anorganische Säurekomponente enthält, ausgewählt aus einer Phosphonsäurekomponente oder einer Sulfonsäurekomponente, ausgewählt ist aus der Gruppe bestehend aus durch die folgenden Formeln repräsentierten Verbindungen: 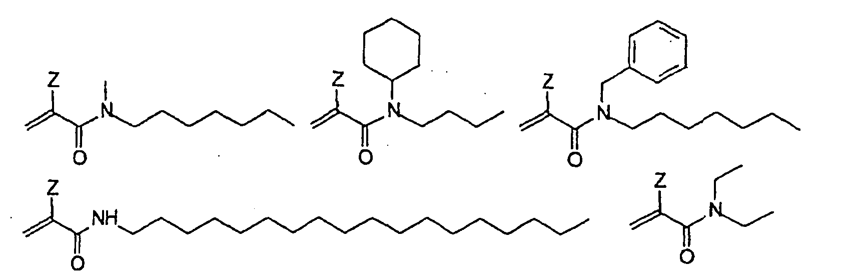 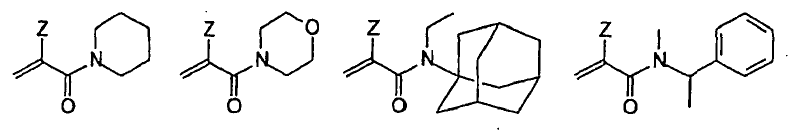 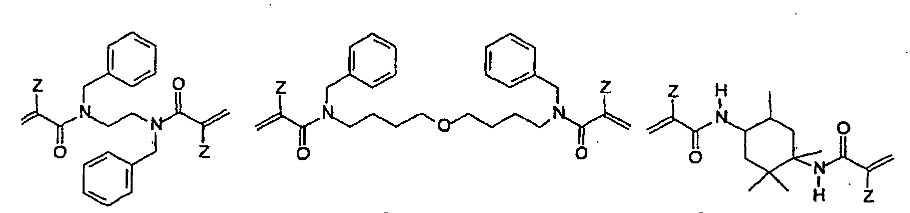 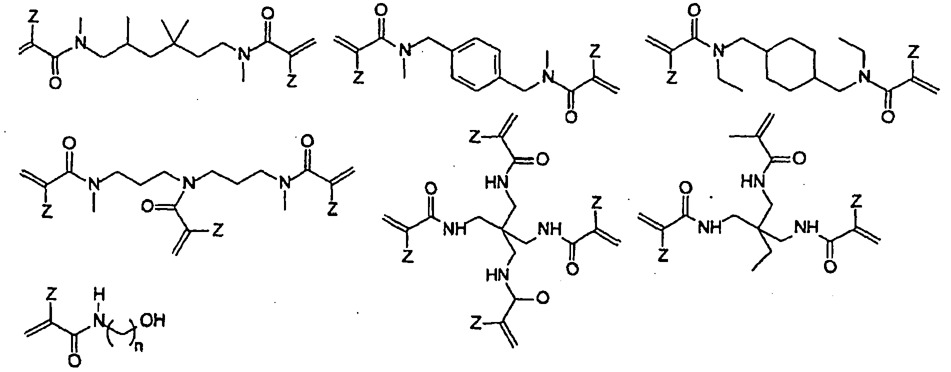 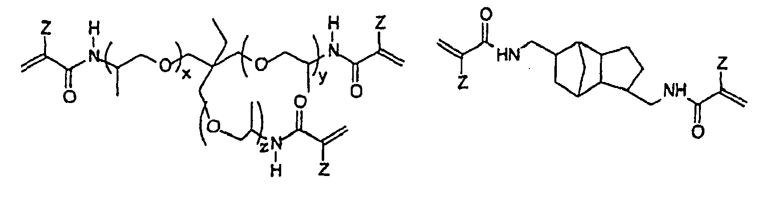 worin bedeuten
n eine ganze Zahl von 2 bis 6;
x, y und z unabhängig von einander eine ganze Zahl von 1 bis 10; und
Z H oder eine substituierte oder unsubstituierte C₁-C₁₈-Alkylgruppe.

12. Zusammensetzung nach einem der vorhergehenden Ansprüche, **dadurch gekennzeichnet, dass** das polymerisierbare N-substituierte Alkylacryl- oder Acrylsäureamidmonomer, das gegebenenfalls eine anorganische Säurekomponente enthält, ausgewählt aus einer Phosphonsäurekomponente oder einer Sulfonsäurekomponente, ausgewählt ist aus (Meth)acrylamidmonomeren.

13. Zusammensetzung nach einem der vorhergehenden Ansprüche, **dadurch gekennzeichnet, dass** das polymerisierbare N-substituierte Alkylacryl- oder Acrylsäureamidmonomer, das mindestens eine anorganische Säurekomponente enthält, ausgewählt ist aus der Gruppe bestehend aus durch die folgenden Formeln repräsentierten Verbindungen: 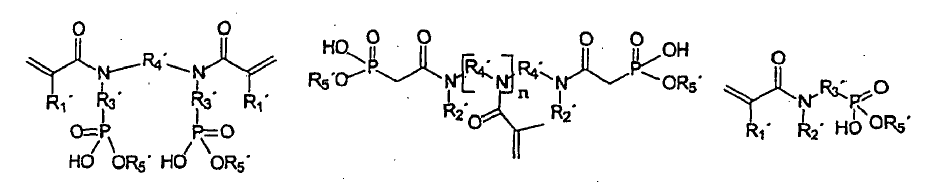 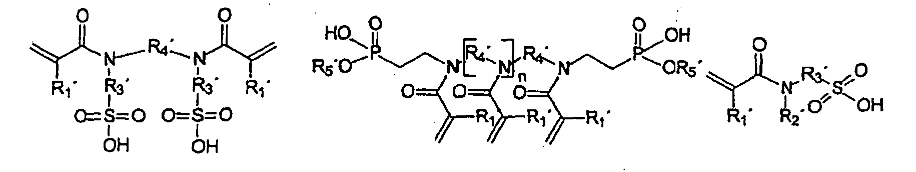 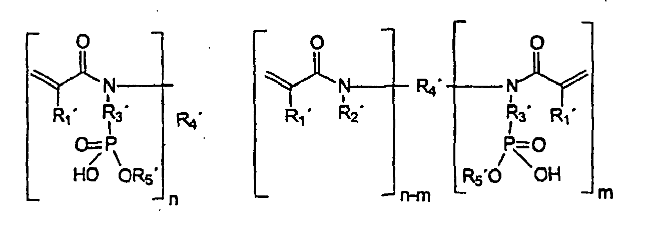 worin bedeuten:
R_{1'} und R_{2'} unabhängig von einander ein Wasserstoffatom oder eine substituierte oder unsubstituierte C₁-C₁₈-Alkylgruppe, eine substituierte oder unsubstituierte Cycloalkylgruppe, eine substituierte oder unsubstituierte C₅-C₁₈-Aryl- oder -Heteroarylgruppe, eine substituierte oder unsubstituierte C₅-C₁₈-Alkylaryl- oder Alkylheteroarylgruppe, eine substituierte oder unsubstituierte C₇-C₃₀-Aralkylgruppe,
R_{3'} und R_{4'} unabhängig von einander eine difunktionelle substituierte oder unsubstituierte C₁-C₁₈-Kohlenstoffkettengruppe, ein difunktionelles substituiertes oder unsubstituiertes Cycloalkylen, eine difunktionelle substituierte oder unsubstituierte C₅-C₁₈-Aryl- oder -Heteroarylgruppe, eine difunktionelle substituierte oder unsubstituierte C₅-C₁₈-Alkylaryl- oder -Alkylheteroarylgruppe, eine difunktionelle substituierte oder unsubstituierte C₇-C₃₀-Aralkylgruppe;
R_{5'} H oder eine substituierte oder unsubstituierte C₁-C₁₈-Alkylgruppe,
n eine ganze Zahl und m eine ganze Zahl von 1 bis 3.

14. Zusammensetzung nach Anspruch 13, **dadurch gekennzeichnet, dass** R_{3'} und R_{4'} unabhängig von einander eine difunktionelle substituierte oder unsubstituierte C₁-C₁₈-Kohlenstoffkettengruppe oder eine difunktionelle substituierte oder unsubstituierte Cycloalkylengruppe bedeuten, die mindestens eine Ether-, Thioether-, Ester-, Thiocarbonyl-, Amid-, Carbonyl-, Sulfonyl-, Urethanbindung oder substituierte oder unsubstituierte Aminbindungen aufweist.

15. Zusammensetzung nach Anspruch 14, **dadurch gekennzeichnet, dass** R_{3'} -(CH₂)₂- oder 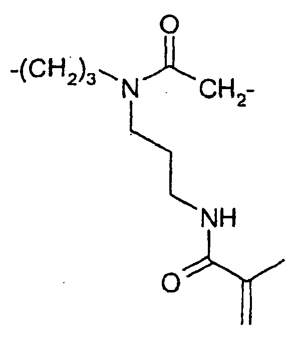 und R_{4'}-(CH₂)₂-O-(CH₂)₂-O-(CH₂)₂- ist.

16. Zusammensetzung nach einem der Ansprüche 1 bis 15, **dadurch gekennzeichnet, dass** das polymerisierbare N-substituierte Alkylacryl- oder Acrylsäureamidmonomer, das mindestens eine anorganische Säurekomponente enthält, ausgewählt ist aus einem der Monomeren gemäß den folgenden Formeln:

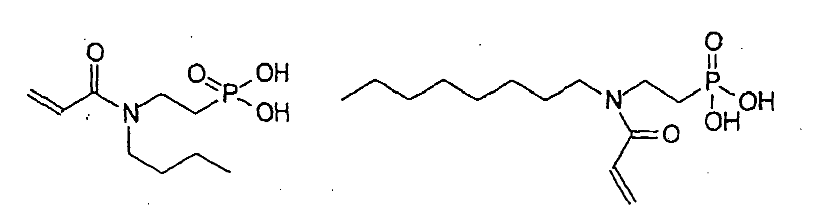

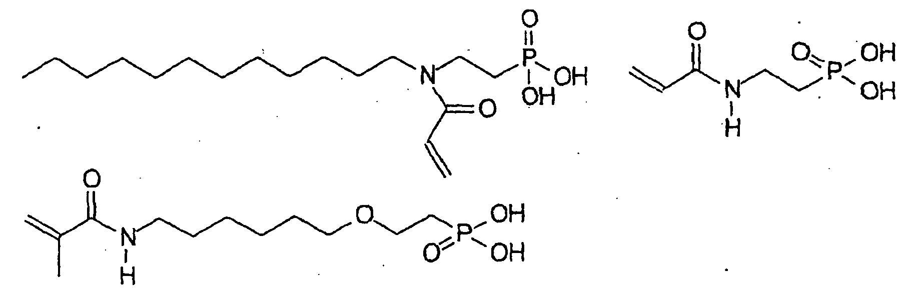

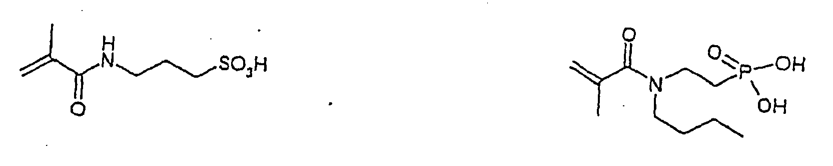

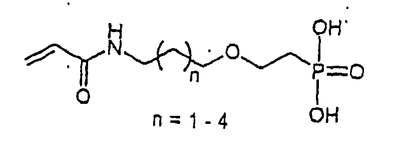

17. Zusammensetzung nach einem der vorhergehenden Ansprüche, **dadurch gekennzeichnet, dass** das polymerisierbare N-substituierte Alkylacryl- oder Acrylsäureamidmonomer, das mindestens eine anorganische Säurekomponente enthält, ausgewählt ist aus (Meth)acrylamidmonomeren.

18. Zusammensetzung nach einem der vorhergehenden Ansprüche, **dadurch gekennzeichnet, dass** das Härtungssystem einen Polymerisationsinitiator, einen Inhibitor oder Stabilisator umfasst.

19. Zusammensetzung nach einem der vorhergehenden Ansprüche, umfassend:
(a) ein polymerisierbares N-substituiertes Alkylacryl- oder Acrylsäureamidmonomer, das mindestens zwei polymerisierbare Einheiten aufweist,
(b) ein polymerisierbares N-substituiertes Alkylacryl- oder Acrylsäureamidmonomer, das mindestens eine anorganische Säurekomponente enthält, und
(c) ein Härtungssystem.

20. Zusammensetzung nach Anspruch 19, worin das polymerisierbare N-substituierte Alkylacryl- oder Acrylsäureamidmonomer, das mindestens zwei polymerisierbare Einheiten aufweist, ausgewählt ist aus der Gruppe bestehend aus durch die folgenden Formeln repräsentierten Verbindungen: 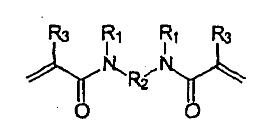 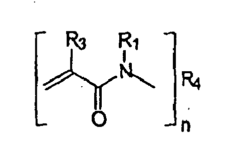 worin
R₁, R_{1'}, R₂, R₃ und R₄ und n die in den Ansprüchen 8 bis 10 definierten Bedeutungen besitzen.

21. Zusammensetzung nach Anspruch 19 oder 20, **dadurch gekennzeichnet, dass** das polymerisierbare N-substituierte Alkylacryl- oder Acrylsäureamidmonomer ausgewählt ist aus der Gruppe bestehend aus durch die folgenden Formeln repräsentierten Verbindungen: 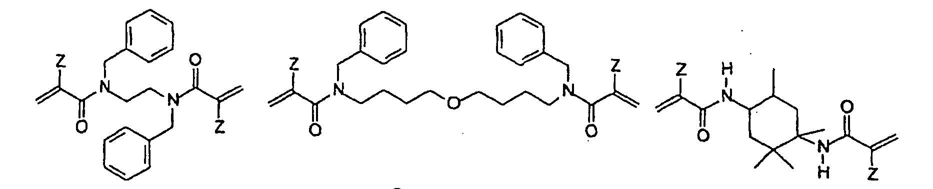 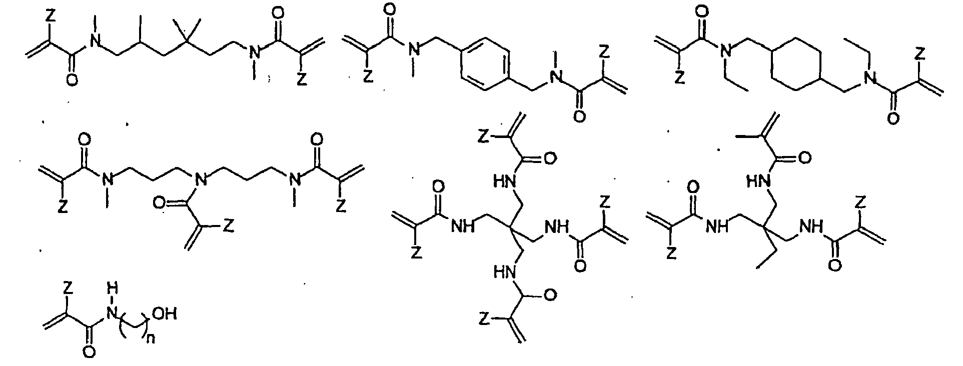 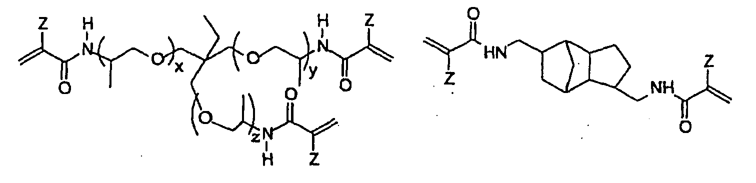 worin Z H oder eine substituierte oder unsubstituierte C₁-C₁₈-Alkylgruppe ist und n, x, y, z die in Anspruch 11 angegebene Bedeutung besitzen.

22. Zusammensetzung nach einem der Ansprüche 18 bis 20, **dadurch gekennzeichnet, dass** das polymerisierbare N-substituierte Alkylacryl- oder Acrylsäureamidmonomer ausgewählt ist aus (Meth)acrylamidmonomeren.

23. Kit, umfassend einen gegen Licht geschützten Behälter, der die Zusammensetzung nach einem der vorhergehenden Ansprüche 1 bis 22 enthält.

24. Kit nach Anspruch 23, der außerdem Verwendungsinstruktionen aufweist.

25. Verfahren zur Herstellung einer selbstätzenden und selbstgrundierenden Dental-Einkomponenten-Kleberzusammensetzung nach einem der vorhergehenden Ansprüche, **gekennzeichnet durch** Mischen von
(A) einem polymerisierbaren N-substituierten Alkylacryl- oder Acrylsäureamidmonomer, das gegebenenfalls eine anorganische Säurekomponente enthält, ausgewählt aus einer Phosphonsäurekomponente oder Sulfonsäurekomponente,
(B) einem Härtungssystem,
(C) einem Wasser-enthaltenden Lösungsmittel und
(D) einem polymerisierbaren N-substituierten Alkylacryl- oder Acrylsäureamidmonomer, das mindestens eine anorganische Säurekomponente enthält, ausgewählt aus einer Phosphonsäurekomponente oder einer Sulfonsäurekomponente.

26. Polymerisierbares N-substituiertes Alkylacryl- oder Acrylsäureamidmonomer, das mindestens eine anorganische Säurekomponente enthält, ausgewählt aus einer Phosphonsäurekomponente oder einer Sulfonsäurekomponente, das ausgewählt ist aus der Gruppe bestehend aus durch die folgenden Formeln repräsentierten Verbindungen: 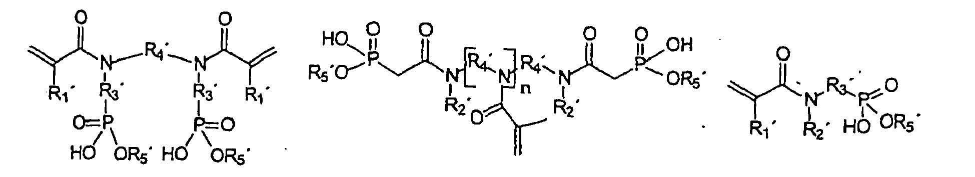 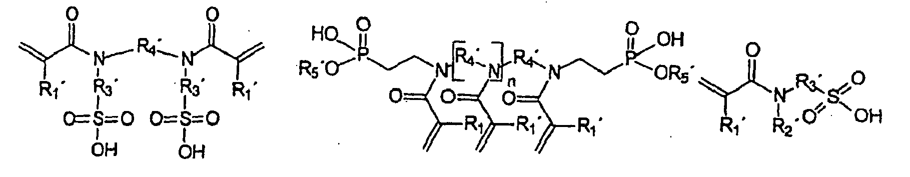 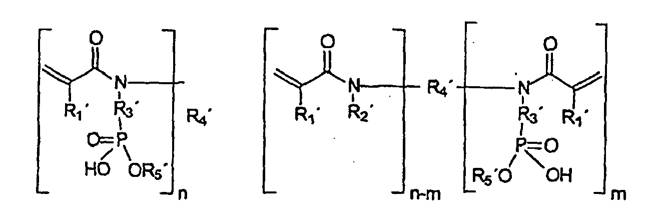 worin R_{1'}, R_{2'}, R_{3'}, R_{4'}, R_{5'}, n und m die in den Ansprüchen 13 bis 15 angegebene Bedeutung besitzen.

27. Verfahren zur Herstellung des polymerisierbaren N-substituierten Alkylacryl- oder Acrylsäureamidmonomers, das mindestens eine anorganische Säurekomponente aufweist, gemäß Anspruch 25, das umfasst
(1) Umsetzen einer Di- oder Polyaminverbindung mit einem Vinylphosphonat oder einem Vinylsulfonsäuresalz;
(2) Umsetzen des in (1) erhaltenen Produkts mit einem Alkylacryloyl- oder Acryloylhalogenid der Formel CH₂=CR_{1'}-CO-Hal, worin R_{1'} die in Anspruch 13 definierte Bedeutung aufweist und Hal ein Halogen ist;
(3) Gegebenenfalls Umsetzen des in (2) erhaltenen Produkts mit einem Trialkylhalogensilan im Falle des Umsetzens eines Vinylphosphonats in Stufe (1); und
(4) Hydrolysieren des in Stufe (2) oder (3) erhaltenen Produkts.

## Revendications

1. Composition aqueuse d'adhésif dentaire monocomposant automordançant et autocollant ayant un pH d'au plus 2, qui comprend :
(i) un monomère polymérisable du type amide d'acide acrylique ou alkylacrylique N-substitué qui contient facultativement un fragment acide minéral choisi parmi un fragment acide phosphonique et un fragment acide sulfonique,
(ii) un système durcisseur, et
(iii) un monomère polymérisable du type amide d'acide acrylique ou alkylacrylique N-substitué contenant au moins un fragment acide minéral choisi parmi un fragment acide phosphonique et un fragment acide sulfonique,
dans laquelle
le monomère polymérisable du type amide d'acide acrylique ou alkylacrylique N-substitué (i) ou
le monomère polymérisable du type amide d'acide acrylique ou alkylacrylique N-substitué contenant au moins un fragment acide minéral (iii) contient au moins deux groupes polymérisables.

2. Composition selon la revendication 1, qui comprend un acide organique ou minéral.

3. Composition selon la revendication 2, dans laquelle l'acide organique est choisi dans le groupe formé par l'acide méthacrylique, l'acide acrylique, l'acide fumarique, l'acide maléique, l'acide citrique, l'acide itaconique et l'acide formique, et dans laquelle l'acide minéral est choisi parmi l'acide phosphorique, l'acide sulfurique et l'acide fluorhydrique.

4. Composition selon l'une quelconque des revendications précédentes, qui contient, en plus de l'eau, un solvant organique hydrosoluble.

5. Composition selon la revendication 4, dans laquelle le solvant organique hydrosoluble est choisi parmi l'éthanol, le propanol, le butanol, l'acétone et la méthyléthylcétone.

6. Composition selon l'une quelconque des revendications précédentes, dans laquelle lesdits au moins deux groupes polymérisables sont reliés directement ou indirectement par une liaison amide.

7. Composition selon l'une quelconque des revendications précédentes, qui contient de plus une nanocharge.

8. Composition selon l'une quelconque des revendications précédentes, dans laquelle le monomère polymérisable du type amide d'acide acrylique ou alkylacrylique N-substitué est choisi dans le groupe formé par les composés représentés par les formules suivantes : 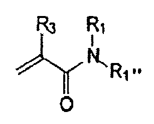 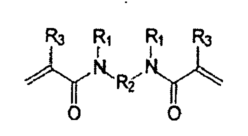 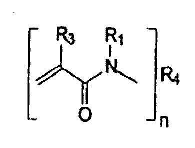 où
R₁, R_{1''} et R₃ sont indépendamment l'hydrogène ou un groupe alkyle en C₁ à C₁₈ substitué ou non substitué, un groupe cycloalkyle substitué ou non substitué, un groupe aryle ou hétéroaryle en C₅ à C₁₈ substitué ou non substitué, un groupe alkylaryle ou alkylhétéroaryle en C₅ à C₁₈ substitué ou non substitué, un groupe aralkyle en C₇ à C₃₀ substitué ou non substitué,
à condition que, dans le cas où l'un de R₁ et R_{1''} est l'hydrogène dans la formule (F1), l'autre ne soit pas l'hydrogène ;
R₂ est un groupe alkylène en C₁ à C₁₈ difonctionnel substitué ou non substitué, un groupe cycloalkylène difonctionnel substitué ou non substitué, un groupe aryle ou hétéroaryle en C₅ à C₁₈ difonctionnel substitué ou non substitué, un groupe alkylaryle ou alkylhétéroaryle en C₅ à C₁₈ difonctionnel substitué ou non substitué, un groupe aralkyle en C₇ à C₃₀ difonctionnel substitué ou non substitué,
R₄ est un groupe à chaîne carbonée en C₁ à C₁₈ mono- ou polyfonctionnel substitué ou non substitué, un groupe cycloalkyle substitué ou non substitué mono- ou polyfonctionnel, un groupe aryle ou hétéroaryle en C₅ à C₁₈ mono- ou polyfonctionnel substitué ou non substitué, un groupe alkylaryle ou alkylhétéroaryle en C₅ à C₁₈ mono- ou polyfonctionnel substitué ou non substitué, un groupe aralkyle en C₇ à C₃₀ mono- ou polyfonctionnel substitué ou non substitué, et
n est un nombre entier.

9. Composition selon la revendication 8, **caractérisée en ce que** R₂ et R₄ sont indépendamment un groupe à chaîne carbonée en C₁ à C₁₈ di- ou polyfonctionnel substitué ou non substitué ou un groupe cycloalkylène di- ou polyfonctionnel substitué ou non substitué, qui a au moins une liaison choisie parmi les liaisons éther, thioéther, ester, thiocarbonyle, amide, carbonyle, sulfonyle, uréthanne et amine substituée ou non substituée.

10. Composition selon la revendication 9, **caractérisée en ce que**
R₂ ou R₄ est - (CH₂)₂-O-(CH₂)₂-O-(CH₂)₂-, 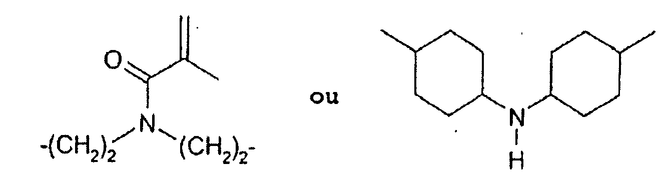 et
n est 2.

11. Composition selon l'une des revendications précédentes, **caractérisée en ce que** le monomère polymérisable du type amide d'acide acrylique ou alkylacrylique N-substitué qui contient facultativement un fragment acide minéral choisi parmi un fragment acide phosphonique et un fragment acide sulfonique est choisi dans le groupe formé par les composés représentés par les formules suivantes : 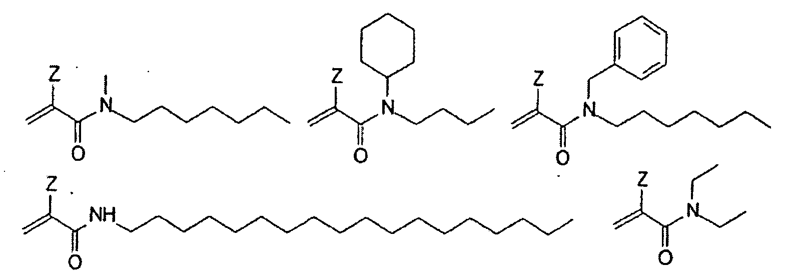 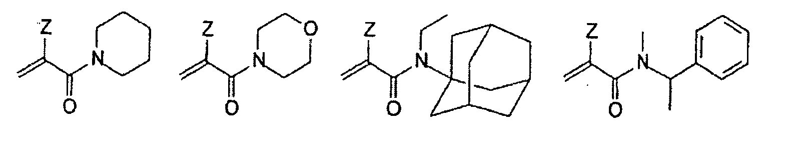 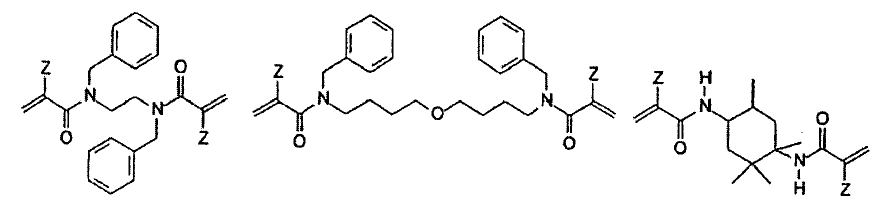 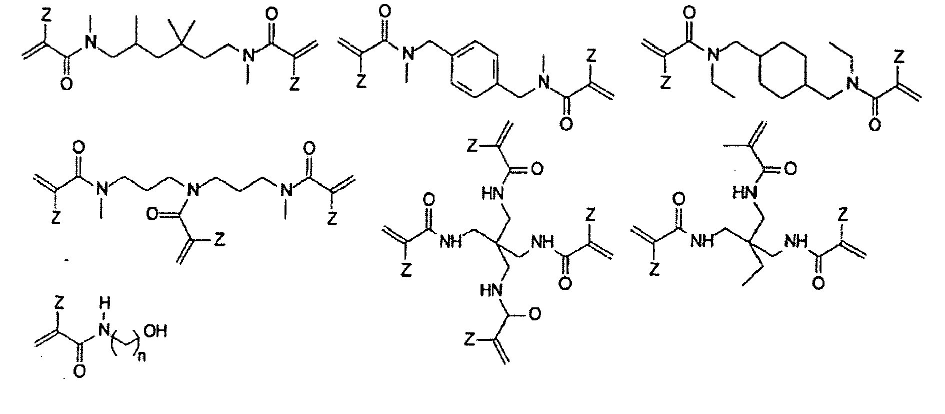 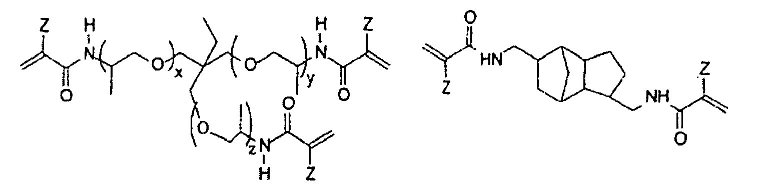 où
n est un nombre entier de 2 à 6 ;
x, y et z sont indépendamment un nombre entier de 1 à 10 ; et
Z est H ou un groupe alkyle en C₁ à C₁₈ substitué ou non substitué.

12. Composition selon l'une des revendications précédentes, **caractérisée en ce que** le monomère polymérisable du type amide d'acide acrylique ou alkylacrylique N-substitué qui contient facultativement un fragment acide minéral choisi parmi un fragment acide phosphonique et un fragment acide sulfonique est choisi parmi les monomères du type (méth)acrylamide.

13. Composition selon l'une des revendications précédentes, **caractérisée en ce que** le monomère polymérisable du type amide d'acide acrylique ou alkylacrylique N-substitué contenant au moins un fragment acide minéral est choisi dans le groupe formé par les composés représentés par les formules suivantes : 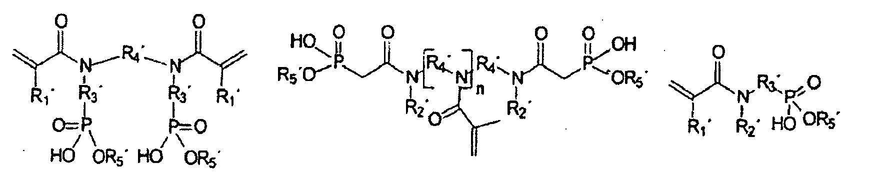 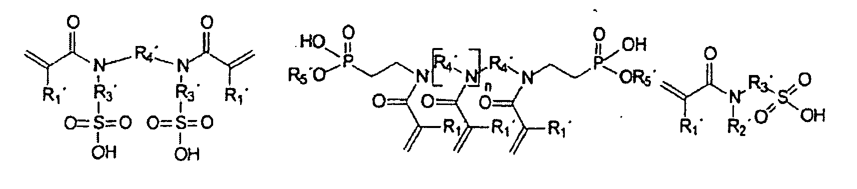 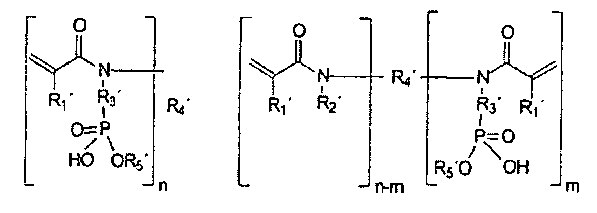 où
R_{1'} et R_{2'} représentent indépendamment l'un de l'autre un atome d'hydrogène ou un groupe alkyle en C₁ à C₁₈ substitué ou non substitué, un groupe cycloalkyle substitué ou non substitué, un groupe aryle ou hétéroaryle en C₅ à C₁₈ substitué ou non substitué, un groupe alkylaryle ou alkylhétéroaryle en C₅ à C₁₈ substitué ou non substitué, un groupe aralkyle en C₇ à C₃₀ substitué ou non substitué,
R_{3'} et R_{4'} représentent indépendamment l'un de l'autre un groupe à chaîne carbonée en C₁ à C₁₈ difonctionnel substitué ou non substitué, un groupe cycloalkylène difonctionnel substitué ou non substitué, un groupe aryle ou hétéroaryle en C₅ à C₁₈ difonctionnel substitué ou non substitué, un groupe alkylaryle ou alkylhétéroaryle en C₅ à C₁₈ difonctionnel substitué ou non substitué, un groupe aralkyle en C₇ à C₃₀ difonctionnel substitué ou non substitué,
R_{5'} représente H ou un groupe alkyle en C₁ à C₁₈ substitué ou non substitué,
n est un nombre entier et
m est un nombre entier de 1 à 3.

14. Composition selon la revendication 13, **caractérisée en ce que** R_{3'} et R_{4'} sont indépendamment l'un de l'autre un groupe à chaîne carbonée en C₁ à C₁₈ difonctionnel substitué ou non substitué ou un groupe cycloalkylène difonctionnel substitué ou non substitué, qui a au moins une liaison choisie parmi les liaisons éther, thioéther, ester, thiocarbonyle, amide, carbonyle, sulfonyle, uréthanne et amine substituée ou non substituée.

15. Composition selon la revendication 14, **caractérisée en ce que**
R_{3'} est -(CH₂)₂- ou 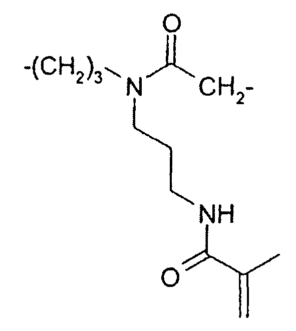 et R_{4'} est -(CH₂)₂-O-(CH₂)₂-O-(CH₂)₂-.

16. Composition selon l'une des revendications 1 à 15, **caractérisée en ce que** le monomère polymérisable du type amide d'acide acrylique ou alkylacrylique N-substitué contenant au moins un fragment acide minéral est choisi parmi l'un des monomères selon les formules suivantes :

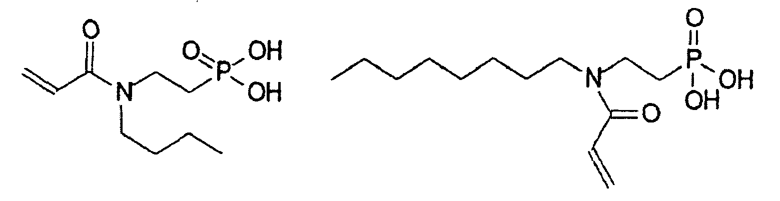

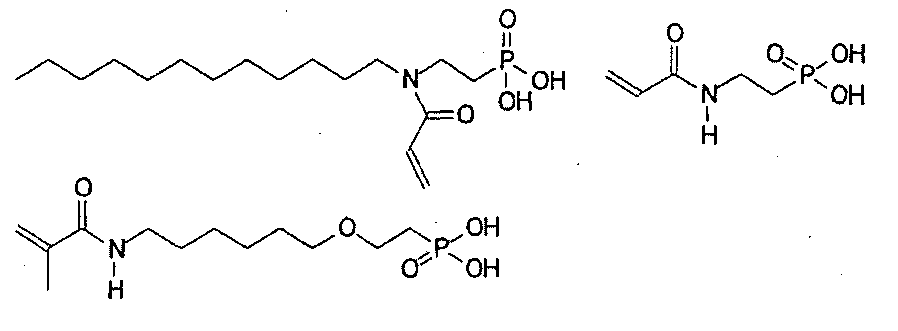

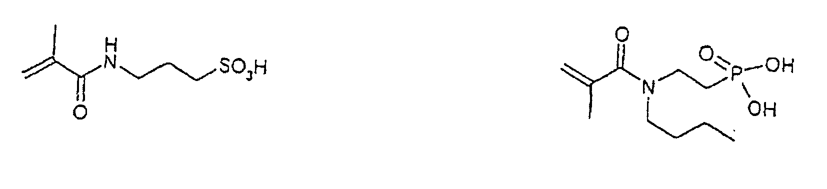

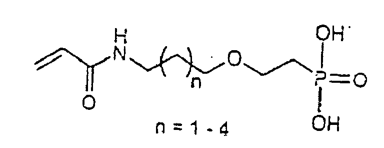

17. Composition selon l'une des revendications précédentes, **caractérisée en ce que** le monomère polymérisable du type amide d'acide acrylique ou alkylacrylique N-substitué contenant au moins un fragment acide minéral est choisi parmi les monomères du type (méth)acrylamide.

18. Composition selon l'une des revendications précédentes, **caractérisée en ce que** le système durcisseur comprend un initiateur de polymérisation, un inhibiteur ou un stabilisant.

19. Composition selon l'une quelconque des revendications précédentes, comprenant :
(a) un monomère polymérisable du type amide d'acide acrylique ou alkylacrylique N-substitué ayant au moins deux fragments polymérisables,
(b) un monomère polymérisable du type amide d'acide acrylique ou alkylacrylique N-substitué contenant au moins un fragment acide minéral, et
(c) un système durcisseur.

20. Composition selon la revendication 19, dans laquelle le monomère polymérisable du type amide d'acide acrylique ou alkylacrylique N-substitué ayant au moins deux fragments polymérisables est choisi dans le groupe formé par les composés représentés par les formules suivantes : 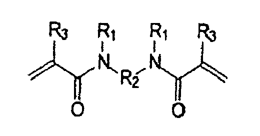 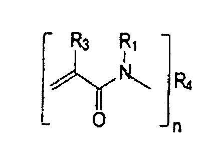 où
R₁, R_{1"}, R₂, R₃ et R₄ et n sont tels que définis dans les revendications 8 à 10.

21. Composition selon la revendication 19 ou 20, **caractérisée en ce que** le monomère polymérisable du type amide d'acide acrylique ou alkylacrylique N-substitué est choisi dans le groupe formé par les composés représentés par les formules suivantes : 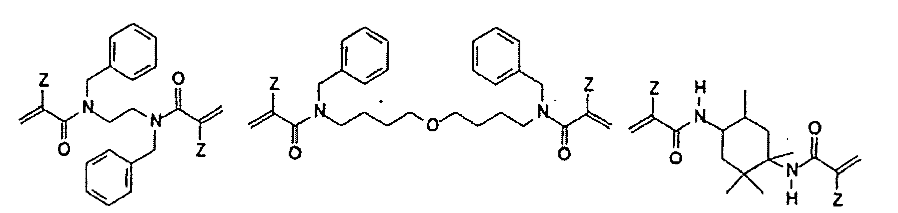 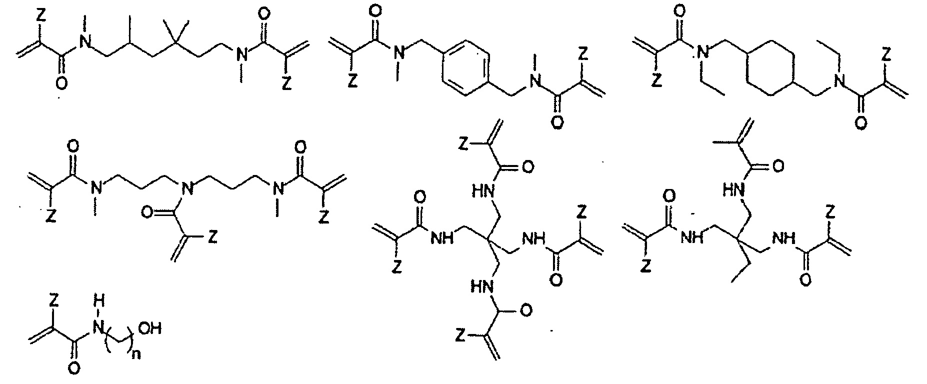 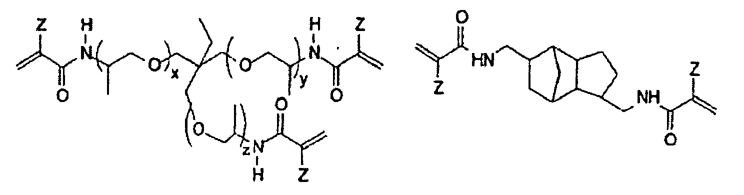 où Z est H ou un groupe alkyle en C₁ à C₁₈ substitué ou non substitué, et n, x, y, z sont tels que définis dans la revendication 11.

22. Composition selon l'une des revendications 18 à 20, **caractérisée en ce que** le monomère polymérisable du type amide d'acide acrylique ou alkylacrylique N-substitué est choisi parmi les monomères du type (méth)acrylamide.

23. Trousse comprenant un récipient protégé de la lumière, qui contient la composition selon l'une des revendications 1 à 22 précédentes.

24. Trousse selon la revendication 23, qui comprend de plus des instructions pour l'emploi.

25. Procédé pour la préparation d'une composition aqueuse d'adhésif dentaire monocomposant automordançant et autocollant selon l'une quelconque des revendications précédentes, **caractérisé en ce qu'**on mélange :
(A) un monomère polymérisable du type amide d'acide acrylique ou alkylacrylique N-substitué qui contient facultativement un fragment acide minéral choisi parmi un fragment acide phosphonique et un fragment acide sulfonique,
(B) un système durcisseur,
(C) un solvant contenant de l'eau, et
(D) un monomère polymérisable du type amide d'acide acrylique ou alkylacrylique N-substitué contenant au moins un fragment acide minéral choisi parmi un fragment acide phosphonique et un fragment acide sulfonique.

26. Monomère polymérisable du type amide d'acide acrylique ou alkylacrylique N-substitué contenant au moins un fragment acide minéral choisi parmi un fragment acide phosphonique et un fragment acide sulfonique, qui est choisi dans le groupe formé par les composés représentés par les formules suivantes : 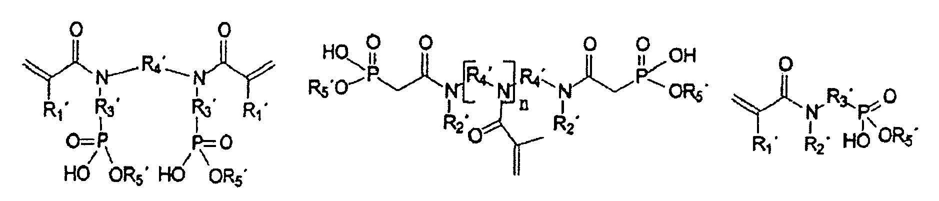 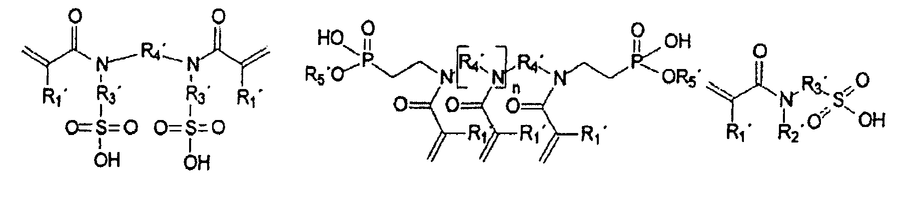 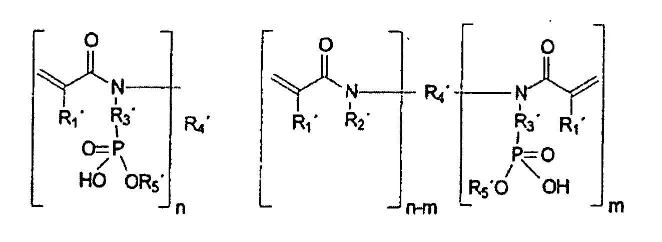 où
R_{1'}, R_{2'}, R_{3'}, R_{4'}, R_{5'}, n et m sont tels que définis dans les revendications 13 à 15.

27. Procédé pour la préparation du monomère polymérisable du type amide d'acide acrylique ou alkylacrylique N-substitué ayant au moins un fragment acide minéral selon la revendication 25, qui comprend
(1) la réaction d'une di- ou polyamine avec un vinylphosphonate ou un sel d'acide vinylsulfonique ;
(2) la réaction du produit obtenu en (1) avec un halogénure d'acryloyle ou d'alkylacryloyle de formule CH₂=CR_{1'}-CO-Hal, où R₁, est tel que défini dans la revendication 13 et Hal est un halogénure ;
(3) facultativement la réaction du produit en (2) avec un trialkyl-halogénosilane dans le cas de la réaction d'un vinylphosphonate dans l'étape (1) ; et
(4) l'hydrolyse du produit obtenu dans l'étape (2) ou (3).
